# NEW EUROPEAN PATENT SPECIFICATION

(11) **EP 1 015 596 B2**
(45) Date of publication and mention of the opposition decision: **14.04.2010**
(45) Mention of the grant of the patent: 30.08.2006
(21) Application number: 98952625.6
(22) Date of filing: 17.09.1998
(51) Int. Cl.: C12N 15/49, C12N 15/62, C07K 14/16, A61K 39/21

(54) **FUSION PROTEINS COMPRISING HIV-1 TAT AND/OR NEF PROTEINS**
HIV-1 TAT UND/ODER NEF ENTHALTENDE FUSIONSPROTEINE
PROTEINES DE FUSION COMPRENANT LES PROTEINES TAT ET/OU NEF DU VIH-1

(30) Priority: 26.09.1997 GB 9720585
(43) Date of publication of application: 05.07.2000
(73) Proprietor: GlaxoSmithKline Biologicals s.a., 1330 Rixensart (BE)
(72) Inventor: BRUCK, Claudine,, Rue de l'Institut 89 B-1330 Rixensart (BE); GODART, Stephane Andre Georges,, Rue de l'Institut 89 B-1330 Rixensart (BE); MARCHAND, Martine,, Rue de l'Institut 89 B-1330 Rixensart (BE)
(74) Representative: Privett, Kathryn Louise
(86) International application number: PCT/EP1998/006040
(87) International publication number: WO 1999/016884

(56) References cited:
- WO-A-94/04686
- BODÉUS M ET AL.: "In vitro binding and phosphorylation of human immunodefciency virus type 1 Nef protein by serine/threonine protein kinase" JOURNAL OF GENERAL VIROLOGY, vol. 76, no. 6, June 1995, pages 1337-1344, XP002092508 READING GB
- SALFELD J ET AL: "A tripartite HIV-1 tat-env-rev fusion protein" EMBO JOURNAL, vol. 9, no. 3, 1 March 1990, pages 965-970, XP000113784
- AHMED A AZAD ET AL: "Large-scale production and characterization of recombinant human immunodeficiency virus type 1 Nef" JOURNAL OF GENERAL VIROLOGY, vol. 75, no. 3, 1 March 1994, pages 651-655, XP000565729
- JANSON H ET AL.: "Protein D, the immunoglobulin D-binding protein of Haemophilus influenzae, is a lipoprotein" INFECTION AND IMMUNITY, vol. 60, no. 4, April 1992, pages 1336-1342, XP002092509 WASHINGTON US cited in the application

## Description

The present invention relates to novel HIV protein constructs, to their use in medicine, to pharmaceutical compositions containing them and to methods of their manufacture.

In particular, the invention relates to fusion proteins comprising HIV-1 Tat and/or Nef proteins.

HIV-1 is the primary cause of the acquired immune deficiency syndrome (AIDS) which is regarded as one of the world's major health problems. Although extensive research throughout the world, has been conducted to produce a vaccine, such efforts thus far, have not been successful.

Non-envelope proteins of HIV-1 have been described and include for example internal structural proteins such as the products of the *gag* and *pol* genes and, other non-structural proteins such as Rev, Nef, Vif and Tat (Greene et al., New England J. Med, 324, 5, 308 et seq (1991) and Bryant et al. (Ed. Pizzo), Pediatr. Infect. Dis. J., 11, 5, 390 et seq (1992).

HIV Nef and Tat proteins are early proteins, that is, they are expressed early in infection and in the absence of structural proteins.

According to the present invention there is provided a protein vaccine composition which comprises an expressed, recovered and isolated protein comprising
(a) an entire HIV Tat protein or Tat with a C terminal histidine tail, or a mutated Tat which has undergone deletion, addition or substitution of one amino acid, or a mutated Tat as defined by SEQ ID NO. 23, linked to either (i) a protein or lipoprotein fusion partner or (ii) an entire HIV Nef protein or Nef with a C-terminal histidine tail, or Nef which has undergone deletion, addition or substitution of one amino acid; or
(b) an entire HIV Nef protein or Nef with a C- terminal histidine tail, or Nef which has undergone deletion, addition or substitution of one amino acid, linked to either (i) a protein or lipoprotein fusion partner or (ii) an entire HIV Tat protein or Tat with a C terminal histidine tail, or a mutated Tat which has undergone deletion, addition or substitution of one amino acid, or a mutated Tat as defined by in SEQ ID NO. 23; or
(c) an entire HIV Nef protein or Nef with a C- terminal histidine tail, or Nef which has undergone deletion, addition or substitution of one amino acid, linked to an entire HIV Tat protein or Tat with a C terminal histidine tail, or a mutated Tat which has undergone deletion, addition or substitution of one amino acid, or a mutated Tat as defined by in SEQ ID NO. 23, and a protein or lipoprotein fusion partner, in admixture with a pharmaceutically acceptable excipient.
By 'fusion partner' is meant any protein sequence that is not Tat or Nef. Preferably the fusion partner is protein D or its' lipidated derivative Lipoprotein D, from Haemophilius influenzac B. In particular, it is preferred that the N-terminal third, i.e. approximately the first 100-130 amino acids are utilised. This is represented herein as Lipo D 1 /3. In a preferred embodiment of the invention the Nef protein or derivative thereof may be linked to the Tat protein or derivative thereof Such Nef-Tat fusions may optionally also be linked to a protein or lipoprotein fusion partner, such as protein D.

The fusion partner is normally linked to the N-terminus of the Nef or Tat protein.

Derivatives encompassed within the present invention include molecules with a C terminal Histidine tail which preferably comprises between 5-10 Histidine residues. Generally, a histidine tail containing n residues is represented herein as His (n). The presence of an histidine (or 'His') tail aids purification. More specifically, the invention provides proteins with the following structure

| | | | | |
|---|---|---|---|---|
| Lipo D 1/3 | - | Nef | - | His (₆) |
| Lipo D 1/3 | - | Nef-Tat | - | His (6) |
| Prot D 1/3 | - | Nef | - | His (₆) |
| Prot D 1/3 | - | Nef-Tat | - | His (6) |
| | | Nef-Tat | - | His (₆) |

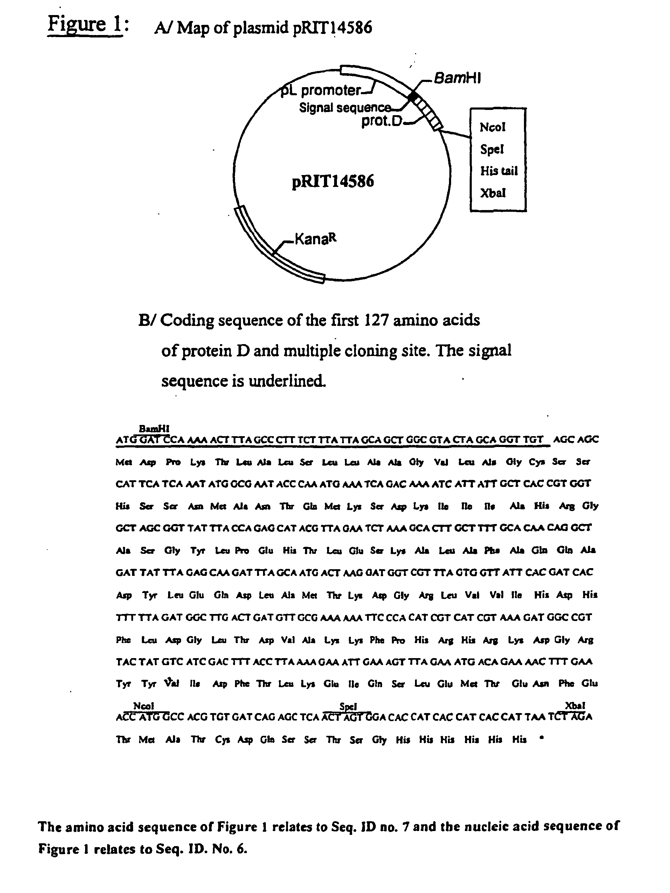

Figure 1 provides the amino-acid (Seq. ID. No. 7) and DNA sequence (Seq. ID. No. 6) of the fusion partner for such constructs.

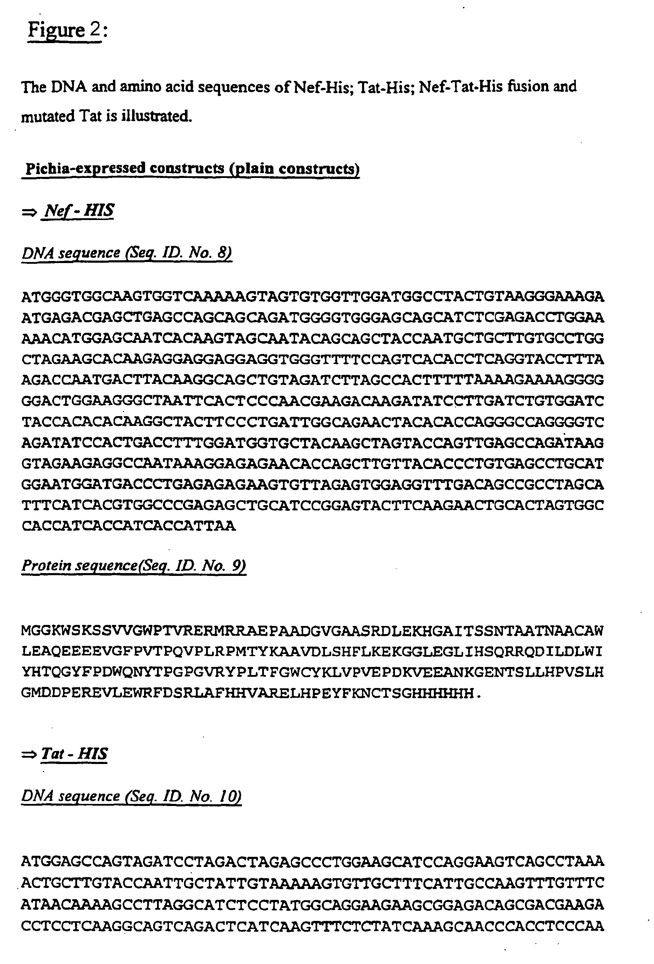

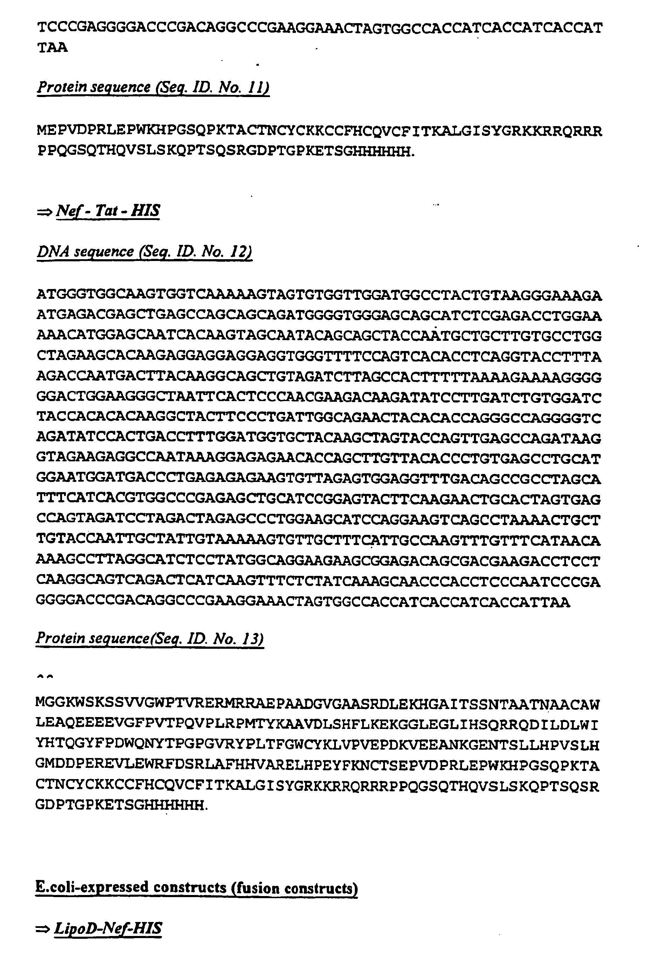

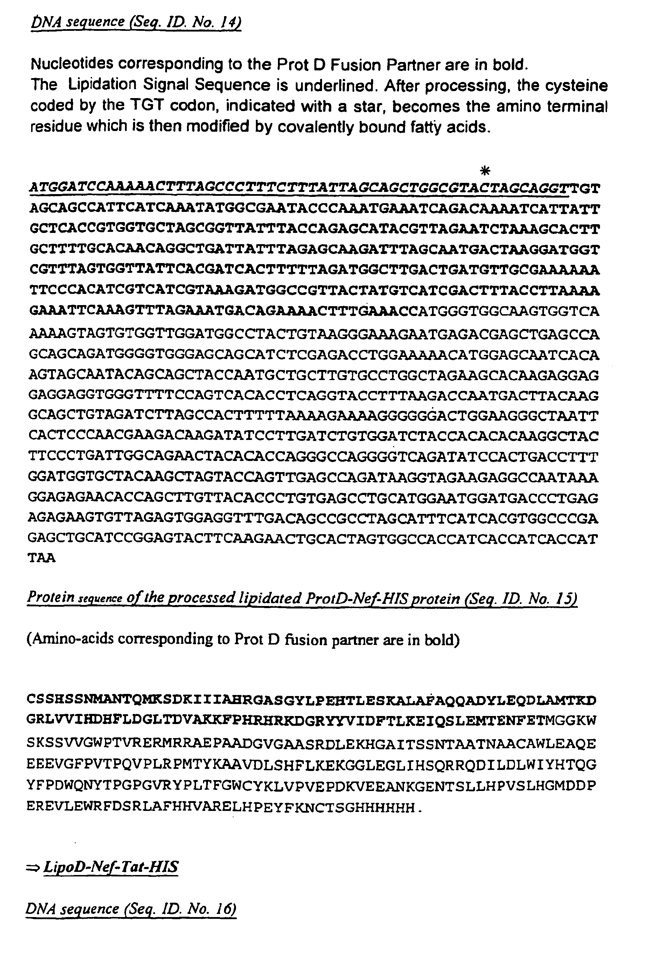

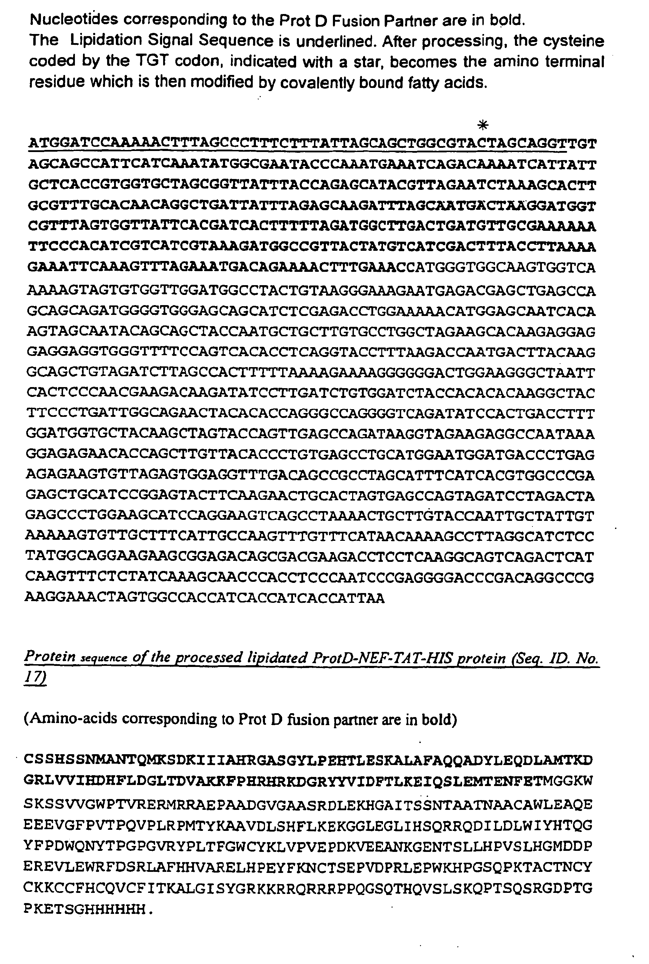

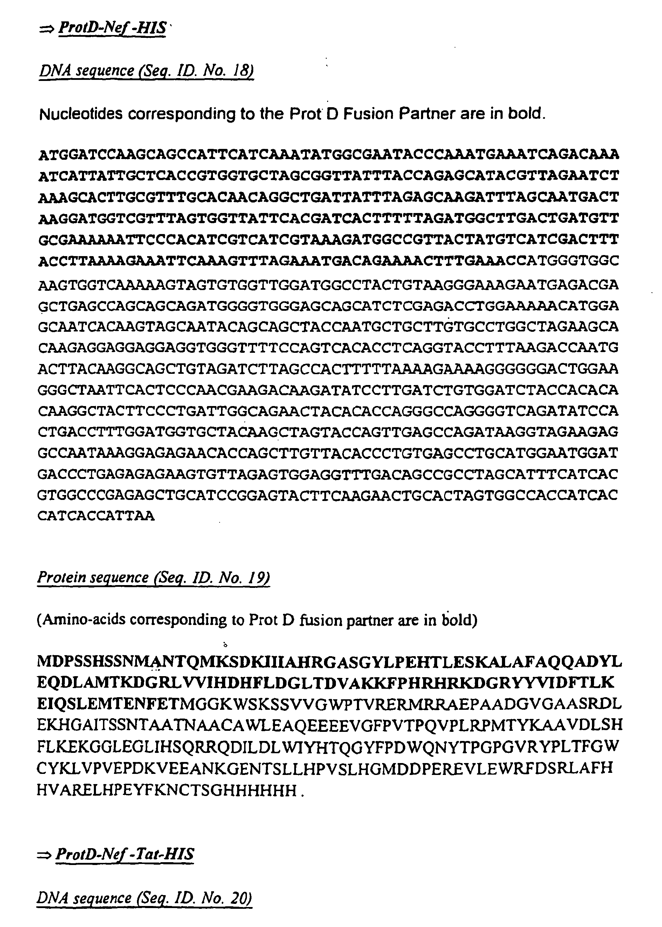

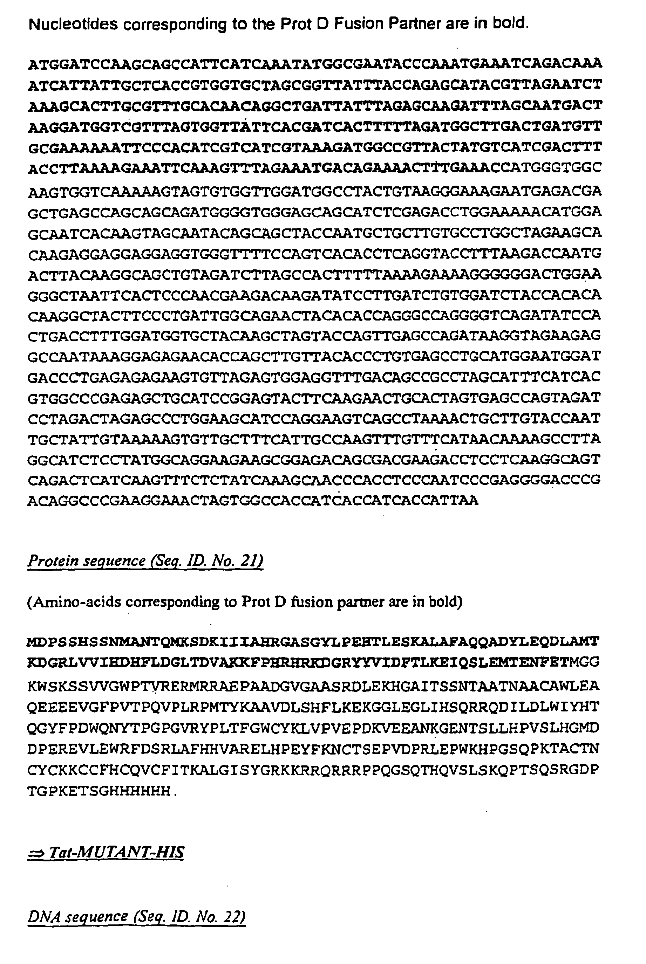

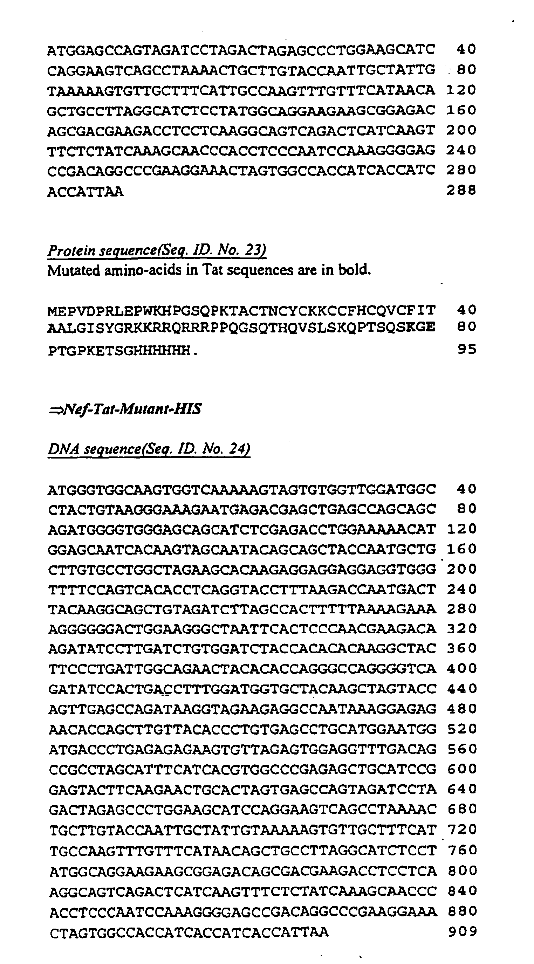

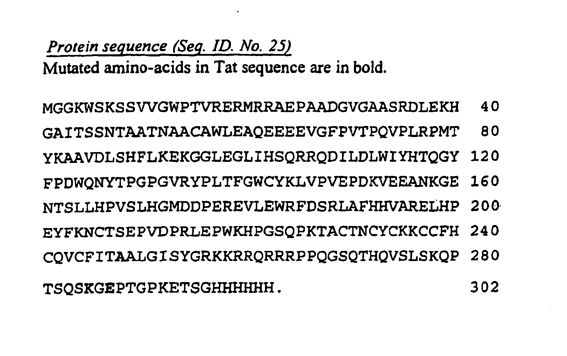

In a preferred embodiment the proteins are expressed with a Histidine tail comprising between 5 to 10 and preferably six Histidine residues. These are advantageous in aiding purification. Separate expression, in yeast (Saccharomyces cerevisiae), of Nef (Macreadie I.G. et al., 1993, Yeast 9 (6) 565-573) and Tat (Braddock M et al., 1989, Cell 58 (2) 269-79) has already been reported Nefprotein only is myristilated. The present invention provides for the first time the expression of Nef and Tat separately in a Pichia expression system (Nef-His and Tat-His constructs), and the successful expression of a fusion construct Nef-Tat-His. The DNA and amino acid sequences of representative Nef-His (Seq. ID. No.s 8 and 9), Tat-His (Seq. ID. No.s 10 and 11)and of Nef-Tat-His fusion proteins (Seq. ID. No.s 12 and 13)are set forth in Figure 2.

Derivatives encompassed within the present invention also include mutated proteins. The term 'mutated' is used herein to mean a molecule which has undergone deletion, addition or substitution of one or more amino acids using well known techniques for site directed mutagenesis or any other conventional method.

A mutated Tat is illustrated in Figure 2 (Seq. ID. No.s 22 and 23) as is a Nef-Tat Mutant-His (Seq. ID. No.s 24 and 25).

A DNA sequence encoding the proteins of the present invention can be synthesized using standard DNA synthesis techniques, such as by enzymatic ligation as described by D.M. Roberts et al. in Biochemistry 1985, 24, 5090-5098, by chemical synthesis, by *in vitro* enzymatic polymerization, or by PCR technology utilising for example a heat stable polymerase, or by a combination of these techniques.

Enzymatic polymerisation of DNA may be carried out *in vitro* using a DNA polymerase such as DNA polymerase I (Klenow fragment) in an appropriate buffer containing the nucleoside triphosphates dATP, dCTP, dGTP and dTTP as required at a temperature of 10°-37°C, generally in a volume of 50µl or less. Enzymatic ligation of DNA fragments may be carried out using a DNA ligase such as T4 DNA ligase in an appropriate buffer, such as 0.05M Tris (pH 7.4), 0.01M MgCl₂, 0.01M dithiothreitol, 1 mM spermidine, 1 mM ATP and 0.1 mg/ml bovine serum albumin, at a temperature of 4°C to ambient, generally in a volume of 50ml or less. The chemical synthesis of the DNA polymer or fragments may be carried out by conventional phosphotriester, phosphite or phosphoramidite chemistry, using solid phase techniques such as those described in 'Chemical and Enzymatic Synthesis of Gene Fragments - A Laboratory Manual' (ed. H.G. Gassen and A. Lang), Verlag Chemie, Weinheim (1982), or in other scientific publications, for example M.J. Gait, H.W.D. Matthes, M. Singh, B.S. Sproat, and R.C. Titmas, Nucleic Acids Research, 1982, 10, 6243; B.S. Sproat, and W. Bannwarth, Tetrahedron Letters, 1983, 24, 5771; M.D. Matteucci and M.H. Caruthers, Tetrahedron Letters, 1980, 21, 719; M.D. Matteucci and M.H. Caruthers, Journal of the American Chemical Society, 1981, 103, 3185; S.P. Adams *et al.,* Journal of the American Chemical Society, 1983, 105, 661; N.D. Sinha, J. Biernat, J. McMannus, and H. Koester, Nucleic Acids Research, 1984, 12,4539; and H.W.D. Matthes *et al.,* EMBO Journal, 1984, 3, 801.

The invention also provides a method of preparing a protein comprising (a) an entire HIV Tat protein or Tat with a C terminal histidine tail, or a mutated Tat which has undergone deletion, addition or substitution of one amino acid, or a mutated Tat as defined by as defined by SEQ ID NO. 23, linked to either (i) a protein or lipoprotein fusion partner or (ii) an entire HIV Nef protein or Nef with a C- terminal histidine tail, or Nef which has undergone deletion, addition or substitution of one amino acid; or (b) an entire HIV Nef protein or Nef with a C- terminal histidine tail, or Nef which has undergone deletion, addition or substitution of one amino acid, linked to either (i) a protein or lipoprotein fusion partner or (ii) an entire HIV Tat protein or Tat with a C terminal histidine tail, or a mutated Tat which has undergone deletion, addition or substitution of one amino acid, or a mutated Tat as defined by SEQ ID NO. 23; or (c) an entire HIV Nef protein or Nef with a C- terminal histidine tail, or Nef which has undergone deletion, addition or substitution of one amino acid, linked to an entire HIV Tat protein or Tat with a C terminal histidine tail, or a mutated Tat which has undergone deletion, addition or substitution of one amino acid, or a mutated Tat as defined by SEQ ID NO. 23, and a protein or lipoprotein fusion partner, in *Pichia pastoris* which method comprises the steps of transforming *Pichia patoris* with DNA encoding said protein, expressing said protein and recovering the protein.

The process of the invention may be performed by conventional recombinant techniques such as described in Maniatis *et al.,* Molecular Cloning - A Laboratory Manual; Cold Spring Harbor, 1982-1989.

The term 'transforming' is used herein to mean the introduction of foreign DNA into a host cell. This can be achieved for example by transformation, transfection or infection with an appropriate plasmid or viral vector using e.g. conventional techniques as described in Genetic Engineering; Eds. S.M. Kingsman and A.J. Kingsman; Blackwell Scientific Publications; Oxford, England, 1988. The term 'transformed' or `transformant' will hereafter apply to the resulting host cell containing and expressing the foreign gene of interest.

The replicable expression vectors may be prepared by cleaving a vector compatible with the host cell to provide a linear DNA segment having an intact replicon, and combining said linear segment with one or more DNA molecules which, together with said linear segment encode the desired product, such as the DNA polymer encoding the protein of the invention, or derivative thereof, under ligating conditions.

Thus, the DNA polymer may be preformed or formed during the construction of the vector, as desired.

The choice of vector will be determined in part by the host cell, which may be prokaryotic or eukaryotic but preferably is *E*. *coli* or yeast. Suitable vectors include plasmids, bacteriophages, cosmids and recombinant viruses.

The preparation of the replicable expression vector may be carried out conventionally with appropriate enzymes for restriction, polymerisation and ligation of the DNA, by procedures described in, for example, Maniatis *et al.* cited above.

The recombinant host cell is prepared by transforming a host cell with a replicable expression vector under transforming conditions. Suitable transforming conditions are conventional and are described in, for example, Maniatis *et al.* cited above, or " DNA Cloning" Vol. II, D.M. Glover ed., IRL Press Ltd, 1985.

The choice of transforming conditions is determined by the host cell. Thus, a bacterial host such as E. coli may be treated with a solution of CaCl₂ (*Cohen et al.,* Proc. Nat. Acad. Sci., 1973, 69, 2110) or with a solution comprising a mixture of RbC1, MnCl₂, potassium acetate and glycerol, and then with 3-[N-morpholino]-propane-sulphonic acid, RbC1 and glycerol. Mammalian cells in culture may be transformed by calcium co-precipitation of the vector DNA onto the cells.

Culturing the transformed host cell under conditions permitting expression of the DNA polymer is carried out conventionally, as described in, for example, Maniatis *et al*. and "DNA Cloning" cited above. Thus, preferably the cell is supplied with nutrient and cultured at a temperature below 50°C.

The product is recovered by conventional methods according to the host cell. Thus, where the host cell is bacterial, such as E. coli - or yeast such as Pichia; it may be lysed physically, chemically or enzymatically and the protein product isolated from the resulting lysate. Where the host cell is mammalian, the product may generally be isolated from the nutrient medium or from cell free extracts. Conventional protein isolation techniques include selective precipitation, adsorption chromatography, and affinity chromatography including a monoclonal antibody affinity column.

For proteins of the present invention provided with Histidine tails, purification can easily be achieved by the use of a metal ion affinity column. In a preferred embodiment, the protein is further purified by subjecting it to cation ion exchange chromatography and/or Gel filtration chromatography. The protein is then sterilised by passing through a 0.22 µm membrane.

The proteins of the invention can then be formulated as a vaccine, or the Histidine residues enzymatically cleaved.

The proteins of the present invention are provided preferably at least 80% pure more preferably 90% pure as visualised by SDS PAGE. Preferably the proteins appear as a single band by SDS PAGE.

The present invention also provides pharmaceutical composition comprising a protein of the present invention in a pharmaceutically acceptable excipient.

Vaccine preparation is generally described in **New Trends and Developments in Vaccines**, Voller *et al*. (eds.), University Park Press, Baltimore, Maryland, 1978. Encapsulation within liposomes is described by Fullerton, US Patent 4,235,877.

The proteins of the present invention are preferably adjuvanted in the vaccine formulation of the invention. Suitable adjuvants include an aluminium salt such as aluminium hydroxide gel (alum) or aluminium phosphate, but may also be a salt of calcium, iron or zinc, or may be an insoluble suspension of acylated tyrosine, or acylated sugars, cationically or anionically derivatised polysaccharides, or polyphosphazenes.

In the formulation of the inventions it is preferred that the adjuvant composition induces a preferential TH1 response. Suitable adjuvant systems include, for example, a combination of monophosphoryl lipid A or derivative thereof, preferably 3-de-O-acylated monophosphoryl lipid A (3D-MPL) together with an aluminium salt.

An enhanced system involves the combination of a monophosphoryl lipid A and a saponin derivative particularly the combination of QS21 and 3D- MPL as disclosed in WO 94/00153, or a less reactogenic composition where the QS21 is quenched with cholesterol as disclosed in WO 96/33739.

A particularly potent adjuvant formulation involving QS21, 3D-MPL & tocopherol in an oil in water emulsion is described in WO 95/17210 and is a preferred formulation.

Accordingly in one embodiment of the present invention there is provided a vaccine comprising a protein according to the invention adjuvanted with a monophosphoryl lipid A or derivative thereof, especially 3D-MPL.

Preferably the vaccine additionally comprises a saponin, more preferably QS21.

Preferably the formulation additional comprises an oil in water emulsion and tocopherol. The present invention also provides a method for producing a vaccine formulation comprising mixing a protein of the present invention together with a pharmaceutically acceptable excipient, such as 3D-MPL.

The vaccine of the present invention may additional comprise further HIV proteins, such as the envelope glycoprotein gp160 or its derivative gp 120.

In another aspect, the invention relates to an HIV Nef or an HIV Tat protein or derivative thereof expressed in *Pichia pastoris.*

The invention will be further described by reference to the following examples:

### EXAMPLES:

### General

Nef and Tat proteins, two regulatory proteins encoded by the human immunodeficiency virus (HIV-1) were produced in *E.coli* and in the methylotrophic yeast *Pichia pastoris.*

The *nef* gene from the Bru/Lai isolate (Cell 40: 9-17, 1985) was selected for these constructs since this gene is among those that are most closely related to the consensus Nef.

The starting material for the Bru/Lai *nef gene* was a 1170bp DNA fragment cloned on the mammalian expression vector pcDNA3 (pcDNA3/*nef*).

The *tat* gene originates from the BH10 molecular clone. This gene was received as an HTLV III cDNA clone named pCV1 and described in Science, 229, p69-73, 1985.

### 1. EXPRESSION OF HIV-1 nef AND tat SEQUENCES IN E.COLI.

Sequences encoding the Nef protein as well as a fusion of *nef* and *tat* sequences were placed in plasmids vectors: pRIT14586 and pRIT14589 (see figure 1).

Nef and the Nef-Tat fusion were produced as fusion proteins using as fusion partner a part of the protein D. Protein D is an immunoglobulin D binding protein exposed at the surface of the gram-negative bacterium *Haemophilus influenzae.*

pRIT14586 contains, under the control of a λPL promoter, a DNA sequence derived from the bacterium *Haemophilus influenzae* which codes for the first 127 amino acids of the protein D (Infect. Immun. 60 : 1336-1342, 1992), immediately followed by a multiple cloning site region plus a DNA sequence coding for one glycine, 6 histidines residues and a stop codon (Fig. 1A).

This vector is designed to express a processed lipidated His tailed fusion protein (LipoD fusion protein). The fusion protein is synthesised as a precursor with an 18 amino acid residues long signal sequence and after processing, the cysteine at position 19 in the precursor molecule becomes the amino terminal residue which is then modified by covalently bound fatty acids (Fig.1B).

pRIT14589 is almost identical to pRIT14586 except that the protD derived sequence starts immediately after the cysteine 19 codon.
Expression from this vector results in a His tailed, non lipidated fusion protein (Prot D fusion protein).

Four constructs were made: LipoD-*nef-*His, LipoD-*nef tat-*His*,* ProtD-nef His, and ProtD-*nef tat-*His*.*

The first two constructs were made using the expression vector pRIT14586, the last two constructs used pRIT14589.

### 1.1 CONSTRUCTION OF THE RECOMBINANT STRAIN ECLD-N1 PRODUCING THE LIPOD-Nef-HIS FUSION PROTEIN.

### 1.1.1 Construction of the lipoD-nef-His expression plasmid pRIT14595

The *nef* gene(Bru/Lai isolate) was amplified by PCR from pcDNA3/Nef plasmid with primers 01 and 02.

The *nefDNA* region amplified starts at nucleotide 8357 and terminates at nucleotide 8971 (Cell, 40: 9-17, 1985).

An NcoI restriction site ( which carries the ATG codon of the *nef* gene*)* was introduced at the 5'end of the PCR fragment while a SpeI site was introduced at the 3' end.

The PCR fragment obtained and the expression plasmid pRIT14586 were both restricted by NcoI and SpeI, purified on an agarose gel, ligated and transformed in the appropriate *E.coli* host cell, strain AR58.This strain is a cryptic λ lysogen derived from N99 that is *gal*E::Tn10, Δ-8 *(chl*D*-pgl),* Δ-H1 (*cro-chlA*), N⁺, and cI857.

The resulting recombinant plasmid received, after verification of the *nef amplified* region by automatic sequencing,(see section 1.1.2 below) the pRIT14595 denomination.

### 1.1.2 Selection of transformants of E. Coli strain AR58 with pRIT14595.

When transformed in AR58 *E.coli* host strain, the recombinant plasmid directs the heat-inducible production of the heterologous protein.

Heat inducible protein production of several recombinant lipoD-Nef-His transformants was analysed by Coomassie Blue stained SDS-PAGE. All the transformants analysed showed an heat inducible heterologous protein production. The abundance of the recombinant Lipo D-Nef-Tat-His fusion protein was estimated at 10% of total protein.

One of the transformants was selected and given the laboratory accession number ECLD-N1.

The recombinant plasmid was reisolated from strain ECLD-N1, and the sequence of the *nef-*His coding region was confirmed by automated sequencing .This plasmid received the official designation pRIT14595.

The fully processed and acylated recombinant Lipo D-*nef*-His fusion protein produced by strain ECLD-N1 is composed of:
°Fatty acids
°109 a.a. of proteinD (starting at a.a.19 and extending to a.a.127).
°A methionine, created by the use of NcoI cloning site of pRIT14586 (Fig. 1).
°205a.a. of Nef protein (starting at a.a.2 and extending to a.a.206).
°A threonine and a serine created by the cloning procedure (cloning at SpeI site of pRIT14586).
°One glycine and six histidines.

### 1.2 CONSTRUCTION OF RECOMBINANT STRAIN ECD-N1 PRODUCING PROT D-Nef-HIS FUSION PROTEIN.

Construction of expression plasmid pRIT14600 encoding the Prot D-Nef-His fusion protein was identical to the plasmid construction described in example 1.1.1 with the exception that pRIT14589 was used as receptor plasmid for the PCR amplified *nef* fragment.

E.coli AR58 strain was transformed with pRIT14600 and transformants were analysed as described in example 1.1.2. The transformant selected received laboratory accession number ECD-N1.

### 1.3 CONSTRUCTION OF RECOMBINANT STRAIN ECLD-NT6 PRODUCING THE LIPO D-Nef-Tat-HIS FUSION PROTEIN.

### 1.3.1 Construction of the lipo D-Nef-Tat-His expression plasmid pRIT14596

The *tat* gene(BH10 isolate) was amplified by PCR from a derivative of the pCV1 plasmid with primers 03 and 04. SpeI restriction sites were introduced at both ends of the PCR fragment.

The nucleotide sequence of the amplified *tat* gene is illustrated in the pCV1 clone (Science 229 : 69-73, 1985) and covers nucleotide 5414 till nucleotide 7998.

The PCR fragment obtained and the plasmid pRIT14595 (expressing lipoD-Nef-His protein) were both digested by SpeI restriction enzyme, purified on an agarose gel, ligated and transformed in competent AR58 cells. The resulting recombinant plasmid received, after verification of the *tat* amplified sequence by automatic sequencing (see section 1.3.2 below), the pRIT14596 denomination.

### 1.3.2 Selection of transformants of strain AR58 with pRIT14596

Transformants were grown, heat induced and their proteins were analysed by Coomassie Blue stained gels. The production level of the recombinant protein was estimated at 1 % of total protein. One recombinant strain was selected and received the laboratory denomination ECLD-NT6.

The lipoD-*nef*-*tat* -His recombinant plasmid was reisolated from ECLD-NT6 strain, sequenced and received the official designation pRIT14596.

The fully processed and acylated recombinant Lipo D-Nef-Tat-His fusion protein produced by strain ECLD-N6 is composed of:
°Fatty acids
°109 a.a. of proteinD (starting at a.a.19 and extending to a.a.127).
°A methionine, created by the use of NcoI cloning site of pRIT14586.
°205a.a. of the Nef protein (starting at a.a.2 and extending to a.a.206)
°A threonine and a serine created by the cloning procedure
°85a.a. of the Tat protein (starting at a.a.2 and extending to a.a.86)
°A threonine and a serine introduced by cloning procedure
°One glycine and six histidines.

### 1.4 CONSTRUCTION OF RECOMBINANT STRAIN ECD-NT1 PRODUCING PROT D-Nef-Tat-HIS FUSION PROTEIN.

Construction of expression plasmid pRIT14601 encoding the Prot D-Nef Tat-His fusion protein was identical to the plasmid construction described in example 1.3.1 with the exception that pRIT14600 was used as receptor plasmid for the PCR amplified *nef* fragment.

*E.coli* AR58 strain was transformed with pRIT14601 and transformants were analysed as described previously. The transformant selected received laboratory accession number ECD-NT1.

### 2. EXPRESSION OF HIV-1 nef AND tat SEQUENCES IN PICHIA PASTORIS.

Nef protein, Tat protein and the fusion Nef-Tat were expressed in the methylotrophic yeast *Pichia pastoris* under the control of the inducible alcohol oxidase (AOX1) promoter.

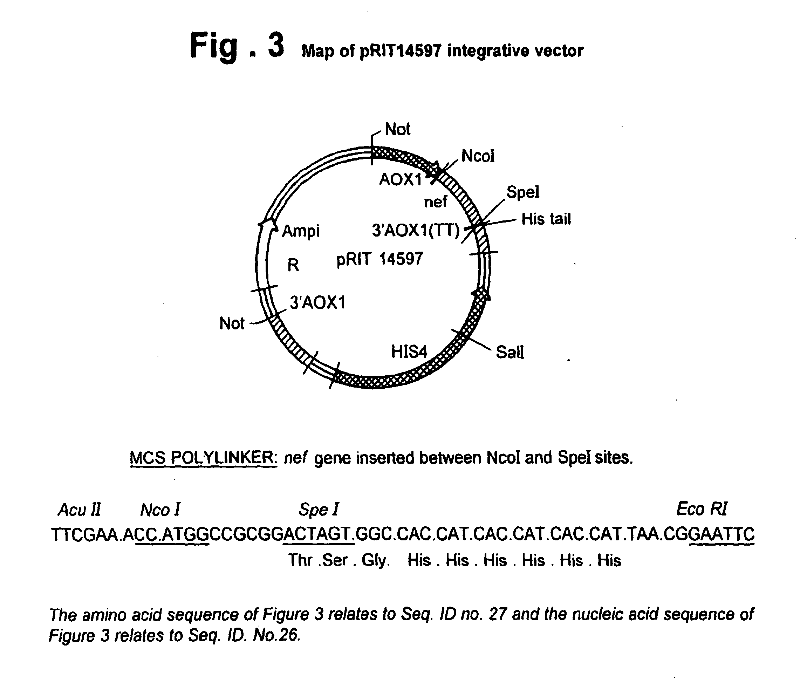

To express these HIV-1 genes a modified version of the integrative vector PHIL-D2 (INVITROGEN) was used. This vector was modified in such a way that expression of heterologous protein starts immediately after the native ATG codon of the AOX1 gene and will produce recombinant protein with a tail of one glycine and six histidines residues. This PHIL-D2-MOD vector was constructed by cloning an oligonucleotide linker between the adjacent AsuII and EcoRI sites of PHIL-D2 vector (see Figure 3). In addition to the His tail, this linker carries NcoI, SpeI and XbaI restriction sites between which *nef, tat* and *nef-tat* fusion were inserted.

### 2.1 CONSTRUCTION OF THE INTEGRATIVE VECTORS pRIT14597 (encoding Nef-His protein), pRIT14598 (encoding Tat-His protein) and pRIT14599 (encoding fusion Nef-Tat-His).

The *nef* gene was amplified by PCR from the pcDNA3/Nef plasmid with primers 01 and 02(see section 1.1.1 construction of pRIT14595).The PCR fragment obtained and the integrative PHIL-D2-MOD vector were both restricted by Ncol and SpeI, purified on agarose gel and ligated to create the integrative plasmid pRIT14597 (see Figure 3).

The *tat* gene was amplified by PCR from a derivative of the pCV 1 plasmid with primers 05 and 04(see section 1.3.1 construction of pRIT14596):

An Ncol restriction site was introduced at the 5' end of the PCR fragment while a Spel site was introduced at the 3' end with primer 04. The PCR fragment obtained and the PHIL-D2-MOD vector were both restricted by Ncol and Spel, purified on agarose gel and ligated to create the integrative plasmid pRIT14598.

To construct pRIT14599, a 910bp DNA fragment corresponding to the *nef-tat-*His coding sequence was ligated between the EcoRI blunted(T4 polymerase) and NcoI sites of the PHIL-D2-MOD vector. The *nef-tat-*His coding fragment was obtained by XbaI blunted(T4 polymerase) and NcoI digestions of pRIT14596.

### 2.2 TRANSFORMATION OF PICHIA PASTORIS STRAIN GS115(his4).

To obtain *Pichia pastoris* strains expressing Nef-His, Tat-His and the fusion Nef-Tat-His, strain GS 115 was transformed with linear NotI fragments carrying the respective expression cassettes plus the HIS4 gene to complement his4 in the host genome. Transformation of GS115 with NotI-linear fragments favors recombination at the AOXI locus.

Multicopy integrant clones were selected by quantitative dot blot analysis and the type of integration, insertion (Mut⁺phenotype) or transplacement (Mut⁵phenotype), was determined.

From each transformation, one transformant showing a high production level for the recombinant protein was selected :

Strain Y1738 (Mut⁺ phenotype) producing the recombinant Nef-His protein, a myristylated 215 amino acids protein which is composed of:
°Myristic acid
°A methionine, created by the use of NcoI cloning site of PHIL-D2-MOD vector
°205 a.a. of Nef protein(starting at a.a.2 and extending to a.a.206)
°A threonine and a serine created by the cloning procedure (cloning at Spel site of PHIL-D2-MOD vector.
°One glycine and six histidines.

Strain Y1739 (Mut⁺ phenotype) producing the Tat-His protein, a 95 amino acid protein which is composed of:
°A methionine created by the use of Ncol cloning site
°85 a.a. of the Tat protein(starting at a.a.2 and extending to a.a.86)
°A threonine and a serine introduced by cloning procedure
°One glycine and six histidines

Strain Y1737(Mut^{s} phenotype) producing the recombinant Nef-Tat-His fusion protein, a myristylated 302 amino acids protein which is composed of:
°Myristic acid
°A methionine, created by the use of NcoI cloning site
°205a.a. of Nef protein(starting at a.a.2 and extending to a.a.206)
°A threonine and a serine created by the cloning procedure
°85a.a. of the Tat protein(starting at a.a.2 and extending to a.a.86)
°A threonine and a serine introduced by the cloning procedure
°One glycine and six histidines

### 3. EXPRESSION OF HIV-1 Tat-MUTANT IN PICHIA PASTORIS

As well as a Nef-Tat mutant fusion protein, a mutant recombinant Tat protein has also been expressed. The mutant Tat protein must be **biologically inactive** while **maintaining** its **immunogenic epitopes.**

A double mutant *tat* gene, constructed by D.Clements (Tulane University) was selected for these constructs.

This *tat* gene (originates from BH10 molecular clone) bears **mutations** in the **active site region (Lys41→Ala**)and in **RGD motif (Arg78→Lys and Asp80→Glu)** ( Virology 235: 48-64, 1997).

The mutant *tat* gene was received as a cDNA fragment subcloned between the EcoRI and HindIII sites within a CMV expression plasmid (pCMVLys41/KGE)

### 3.1 CONSTRUCTION OF THE INTEGRATIVE VECTORS pRIT14912(encoding Tat mutant-His protein) and pRIT14913(encoding fusion Nef-Tat mutant-His).

The *tat* mutant gene was amplified by PCR from the pCMVLys41/KGE plasmid with primers 05 and 04 (see section 2.1construction of pRIT14598)

An NcoI restriction site was introduced at the 5' end of the PCR fragment while a SpeI site was introduced at the 3' end with primer 04. The PCR fragment obtained and the PHIL-D2-MOD vector were both restricted by NcoI and SpeI, purified on agarose gel and ligated to create the integrative plasmid pRIT14912

To construct pRIT14913, the *tat* mutant gene was amplified by PCR from the pCMVLys41/KGE plasmid with primers 03 and 04 (see section 1.3.1 construction of pRIT14596).

The PCR fragment obtained and the plasmid pRIT14597 (expressing Nef-His protein) were both digested by Spel restriction enzyme, purified on agarose gel and ligated to create the integrative plasmid pRIT14913

### 3.2 TRANSFORMATION OF PICHIA PASTORIS STRAIN GS115.

Pichia pastoris strains expressing Tat mutant-His protein and the fusion Nef-Tat mutant-His were obtained, by applying integration and recombinant strain selection strategies previously described in section 2.2 .

Two recombinant strains producing Tat mutant-His protein ,a 95 amino-acids protein, were selected: Y1775 (Mut⁺ phenotype) and Y1776(Mut^{s} phenotype).

One recombinant strain expressing Nef-Tat mutant-His fusion protein, a 302 amino-acids protein was selected: Y1774(Mut⁺ phenotype).

### 4. PURIFICATION OF Nef-Tat-His FUSION PROTEIN (PICHIA PASTORIS)

The purification scheme has been developed from 146g of recombinant Pichia pastoris cells (wet weight) or 2L Dyno-mill homogenate OD 55. The chromatographic steps are performed at room temperature. Between steps , Nef-Tat positive fractions are kept overnight in the cold room (+4°C) ; for longer time, samples are frozen at -20°C.

| | | |
|---|---|---|
| 146g of Pichia pastoris cells | | |
| ↓ | | |
| Homogenization | Buffer: 2L 50 mM PO₄ pH 7.0 final OD:50 | |
| ↓ | | |
| Dyno-mill disruption (4 passes) | | |
| ↓ | | |
| Centrifugation | JA 10 rotor / 9500 rpm/ 30 min / room temperature | |
| ↓ | | |
| Dyno-mill Pellet | | |
| ↓ | | |
| Wash | Buffer: +2L 10 mM PO₄ pH 7.5 - | |
| (1h - 4°C) | 150mM - NaCl 0,5% empigen | |
| ↓ | | |
| Centrifugation | JA10 rotor / 9500 rpm/ 30 min / room | |
| ↓ | temperature | |
| Pellet | | |
| ↓ | | |
| Solubilisation | Buffer:+ 660ml 10 mM PO₄ pH 7.5 - | |
| (O/N - 4°C) | 150mM NaCl - 4.0M GuHCl | |
| ↓ | | |
| Reduction | + 0,2M 2-mercaptoethanesulfonic acid, | |
| (4H - room temperature - in the dark) | sodium salt (powder addition) / pH adjusted to 7.5 (with 0,5M NaOH solution) before incubation | |
| ↓ | | |
| Carboxymethylation | + 0,25M Iodoacetamid (powder addition) | |
| (1/2 h - room temperature - in the dark) | / PH adjusted to 7.5 (with O,5M NaOH | |
| | solution) before incubation | |
| ↓ | | |
| Immobilized metal ion affinity chromatography on Ni⁺⁺-NTA-Agarose (Qiagen - 30 ml of resin) | Equilibration buffer: 10 mM PO₄ pH 7.5 -150mM NaCl - 4.0M GuHCl | |
| | Washing buffer: | 1) Equilibration |
| | buffer | |
| | | 2) 10 mM PO₄ pH |
| | 7.5 - 150mM | NaCl - 6M Urea |
| | | 3) 10 mM PO₄ pH |
| | 7.5 - 150mM | NaCl - 6M Urea - 25 |
| | mM | Imidazol |
| | Elution buffer: 10 mM PO₄ pH 7.5 -150mM NaCl - 6M Urea - 0,5M Imidazol | |
| ↓ | | |
| Dilution | Down to an ionic strength of 18 mS/cm² | |
| | Dilution buffer: 10 mM PO₄ pH 7.5 - 6M Urea | |
| ↓ | | |
| Cation exchange chromatography on SP Sepharose FF | Equilibration buffer: 10 mM PO₄ pH 7.5 | |
| (Pharmacia - 30 ml of resin) | - 150mM NaCl - 6.OM Urea | |
| | Washing buffer: | 1) Equilibration |
| | buffer | |
| | | 2) 10 mM PO₄ pH |
| | 7.5 - 250mM | NaCl - 6M Urea |
| | Elution buffer: 10 mM Borate pH 9.0 - | |
| | 2M NaCl - 6M Urea | |
| ↓ | | |
| Concentration | up to 5 mg/ml | |
| | 10kDa Omega membrane(Filtron) | |
| ↓ | | |
| Gel filtration chromatography on Superdex200 XK | Elution buffer: 10 mM PO₄ pH 7.5- | |
| 16/60 | 150mM NaCl - 6M Urea | |
| (Pharmacia - 120 ml of resin) | 5 ml of sample / injection → 5 injections | |
| ↓ | | |
| Dialysis | Buffer: 10 mM PO₄ pH 6.8 - 150mM | |
| (O/N - 4°C) | NaCl- 0,5M Arginin* | |
| ↓ | | |
| Sterile filtration | Millex GV 0,22µm | |

| | | |
|---|---|---|
| * ratio: 0,5M Arginin for a protein concentration of 1600µg/ml. | | |

### Purity

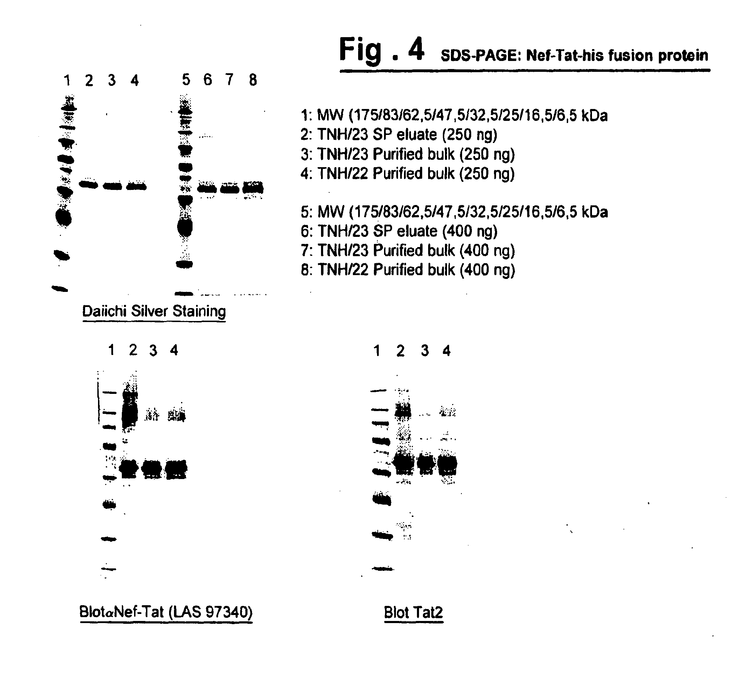

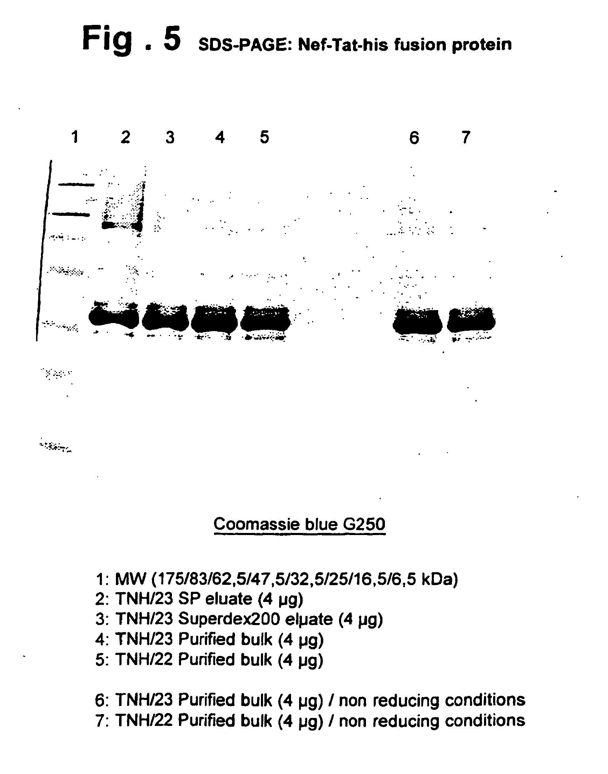

The level of purity as estimated by SDS-PAGE is shown in Figure 4 by Daiichi Silver Staining and in Figure 5 by Coomassie blue G250.

| | |
|---|---|
| After Superdex200 step: | > 95% |
| After dialysis and sterile filtration steps: | > 95% |

### Recovery

51mg of Nef-Tat-his protein are purified from 146g of recombinant Pichia pastoris cells (= 2L of Dyno-mill homogenate OD 55)

### 5. VACCINE PREPARATION

A vaccine prepared in accordance with the invention comprises the expression product of a DNA recombinant encoding an antigen as exemplified in example 1 or 2 and as adjuvant, the formulation comprising a mixture of 3 de -O-acylated monophosphoryl lipid A 3D-MPL and QS21 in an oil/water emulsion.

**3D-MPL:** is a chemically detoxified form of the lipopolysaccharide (LPS) of the Gram-negative bacteria Salmonella minnesota.

Experiments performed at Smith Kline Beecham Biologicals have shown that 3D-MPL combined with various vehicles strongly enhances both the humoral and a TH1 type of cellular immunity.

**QS21:** is one saponin purified from a crude extract of the bark of the Quillaja Saponaria Molina tree, which has a strong adjuvant activity: it activates both antigen-specific lymphoproliferation and CTLs to several antigens.
Experiments performed at Smith Kline Beecham Biologicals have demonstrated a clear synergistic effect of combinations of 3D-MPL and QS21 in the induction of both humoral and TH 1 type cellular immune responses.

**The oil/water emulsion** is composed of 2 oils (a tocopherol and squalene), and of PBS containing Tween 80 as emulsifier. The emulsion comprised 5% squalene 5% tocopherol 0.4% Tween 80 and had an average particle size of 180 nm (see WO 95/17210).

Experiments performed at Smith Kline Beecham Biologicals have proven that the adjunction of this O/W emulsion to 3D-MPL/QS21 further increases their immunostimulant properties.

### Preparation of the oil/water emulsion (2 fold concentrate)

Tween 80 is dissolved in phosphate buffered saline (PBS) to give a 2% solution in the PBS. To provide 100ml two fold concentrate emulsion 5g of DL alpha tocopherol and 5ml of squalene are vortexed to mix thoroughly. 90ml of PBS/Tween solution is added and mixed thoroughly. The resulting emulsion is then passed through a syringe and finally microfluidised by using an M110S microfluidics machine. The resulting oil droplets have a size of approximately 180 nm.

### Preparation of oil in water formulation.

Antigen prepared in accordance with example 1 or 2 (5µg) was diluted in 10 fold concentrated PBS pH 6.8 and H₂O before consecutive addition of SB62, 3D-MPL (5µg), QS21 (5µg) and 50 µg/ml thiomersal as preservative at 5 min interval. The emulsion volume is equal to 50% of the total volume (50µl for a dose of 100µl).

All incubations were carried out at room temperature with agitation.

### 6. IMMUNOGENICITY OF Tat AND Nef-Tat IN RODENTS

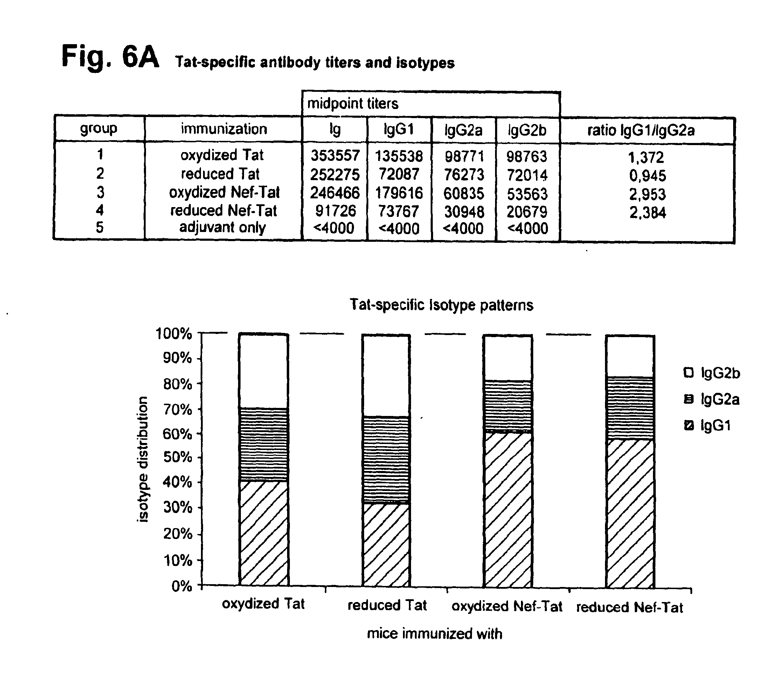

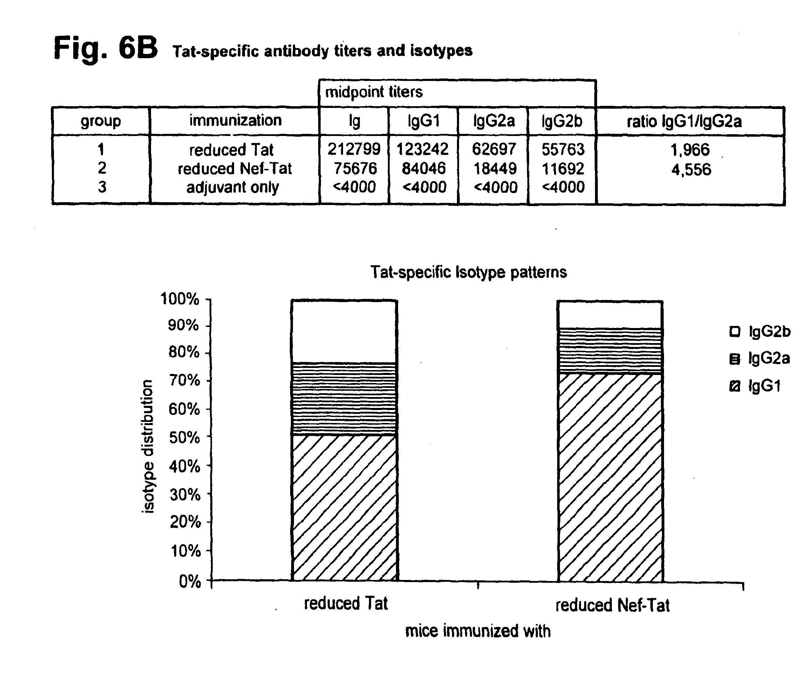

Characterization of the immune response induced after immunization with Tat and NefTat was carried out. To obtain information on isotype profiles and cell-mediated immunity (CMI) two immunization experiments in mice were conducted. In the first experiment mice were immunized twice two weeks apart into the footpad with Tat or NefTat in the oxydized or reduced form, respectively. Antigens were formulated in an oil in water emulsion comprising squalene, tween 80™ (polyoxyethylene sorbitan monooleate) QS21, 3D-MPL and α-tocopherol, and a control group received the adjuvant alone. Two weeks after the last immunization sera were obtained and subjected to Tat-specific ELISA (using reduced Tat for coating) for the determination of antibody titers and isotypes (Figure 6a). The antibody titers were highest in the mice having received oxydized Tat. In general, the oxydized molecules induced higher antibody titers than the reduced forms, and Tat alone induced higher antibody titers than NefTat. The latter observation was confirmed in the second experiment. Most interestingly, the isotype profile of Tat-specific antibodies differed depending on the antigens used for immunization. Tat alone elicited a balanced IgGland IgG2a profile, while Neffat induced a much stronger T_{H2} bias (Figure 6b). This was again confirmed in the second experiment.

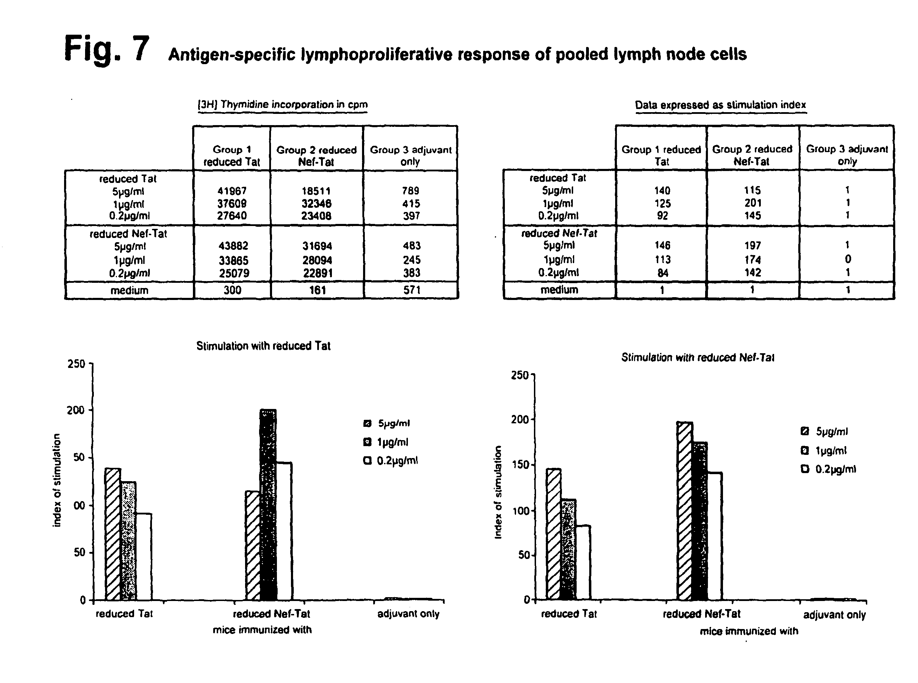

In the second mouse experiment animals received only the reduced forms of the molecules or the adjuvant alone. Besides serological analysis (see above) lymphoproliferative responses from lymph node cells were evaluated. After restimulation of those cells in vitro with Tat or NefTat ³H-thymidine incorporation was measured after 4 days of culture. Presentation of the results as stimulation indices indicates that very strong responses were induced in both groups of mice having received antigen (Figure 7).

In conclusion, the mice studies indicate that Tat as well as Nef-Tat are highly immunogenic candidate vaccine antigens. The immune response directed against the two molecules is characterized by high antibody responses with at least 50% IgG1. Furthermore, strong CMI responses (as measured by lymphoproliferation) were observed.

### 7. FUNCTIONAL PROPERTIES OF THE Tat AND Nef-Tat PROTEINS

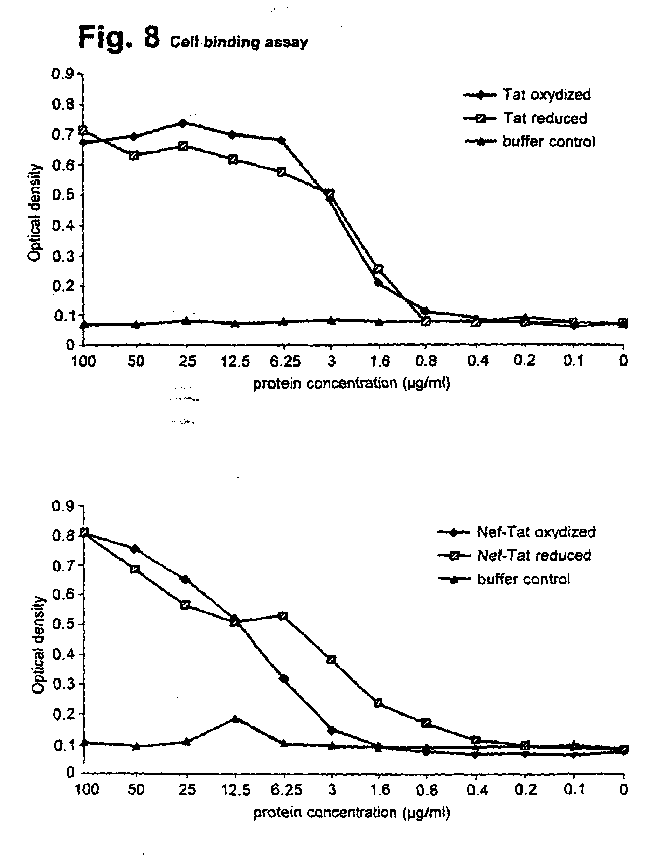

The Tat and NefTat molecules in oxydized or reduced form were investigated for their ability to bind to human T cell lines. Furthermore, the effect on growth of those cell lines was assessed. ELISA plates were coated overnight with different concentration of the Tat and NefTat proteins, the irrelevant gD from herpes simplex virus type II, or with a buffer control alone. After removal of the coating solution HUT-78 cells were added to the wells. After two hours of incubation the wells were washed and binding of cells to the bottom of the wells was assessed microscopically. As a quantitative measure cells were stained with toluidine blue, lysed by SDS, and the toluidine blue concentration in the supernatant was determined with an ELISA plate reader. The results indicate that all four proteins, Tat and NefTat in oxydized or reduced form mediated binding of the cells to the ELISA plate (Figure 8). The irrelevant protein (data not shown) and the buffer did not fix the cells. This indicates that the recombinantly expressed Tat-containing proteins bind specifically to human T cell lines.

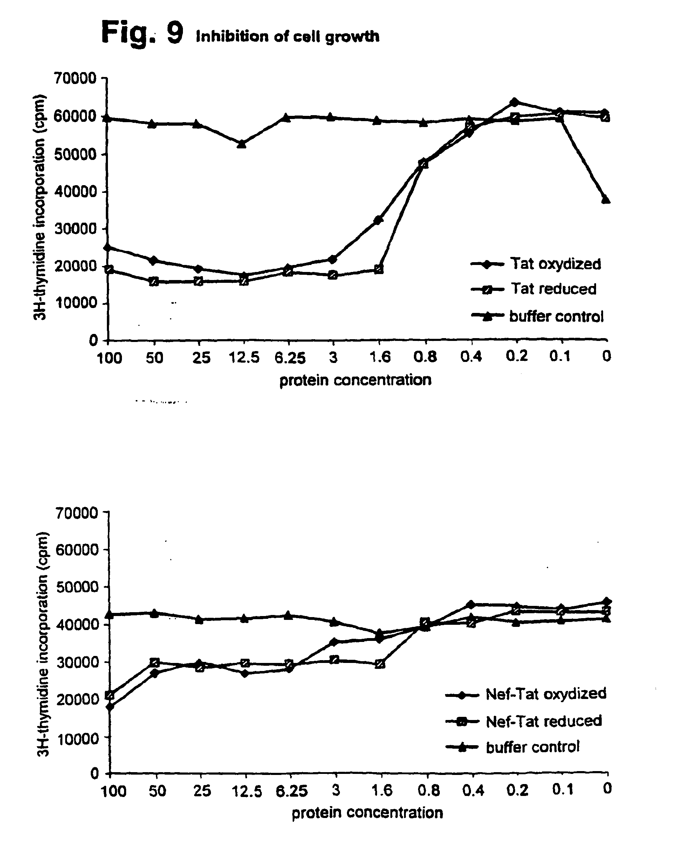

In a second experiment HUT-78 cells were left in contact with the proteins for 16 hours. At the end of the incubation period the cells were labeled with [³H]-thymidine and the incorporation rate was determined as a measure of cell growth. All four proteins included in this assay inhibited cell growth as judged by diminished radioactivity incorporation (Figure 9). The buffer control did not mediate this effect. These results demonstrate that the recombinant Tat-containing proteins are capable of inhibiting growth of a human T cell line.

In summary the functional characterization of the Tat and NefTat proteins reveals that these proteins are able to bind to human Tcell lines. Furthermore, the proteins are able to inhibit growth of such cell lines.

## Claims

1. A protein vaccine composition which comprises an expressed, recovered and isolated protein comprising
(a) an entire HIV Tat protein or Tat with a C terminal histidine tail, or a mutated Tat which has undergone deletion, addition or substitution of one amino acid, or a mutated Tat as defined by SEQ ID NO. 23, linked to either (i) a protein or lipoprotein fusion partner or (ii) an entire HIV Nef protein or Nef with a C-terminal histidine tail, or Nef which has undergone deletion, addition or substitution of one amino acid; or
(b) an entire HIV Nef protein or Nef with a C- terminal histidine tail, or Nef which has undergone deletion, addition or substitution of one amino acid, linked to either (i) a protein or lipoprotein fusion partner or (ii) an entire HIV Tat protein or Tat with a C terminal histidine tail, or a mutated Tat which has undergone deletion, addition or substitution of one amino acid, or a mutated Tat as defined by in SEQ ID NO. 23; or
(c) an entire HIV Nef protein or Nef with a C- terminal histidine tail, or Nef which has undergone deletion, addition or substitution of one amino acid, linked to an entire HIV Tat protein or Tat with a C terminal histidine tail, or a mutated Tat which has undergone deletion, addition or substitution of one amino acid, or a mutated Tat as defined by in SEQ ID NO. 23, and a protein or lipoprotein fusion partner, in admixture with a pharmaceutically acceptable excipient.
in admixture with a pharmaceutically acceptable excipient

2. A composition as claimed in claim 1 comprising a Tat-Nef fusion protein or derivative thereof.

3. A composition as claimed in claim 1 comprising a Nef-Tat fusion protein or derivative thereof.

4. A composition as claimed in any one of claims 1 to 3 wherein the lipoprotein is Haemophilus Influenza B protein D or derivative thereof.

5. A composition as claimed in claim 4 wherein the fusion partner comprises between 100-130 amino acid from the N terminal of Haemophilus Influenza B protein

6. A composition as claimed in any one of Claims 1 to 5, wherein the Tat protein is fused to an HIV Nef protein and a fusion partner.

7. A composition as claimed in any one of claims 1 to 6, wherein the protein has a Histidine tail.

8. A composition as claimed in any one of claims 1 to 7 wherein the protein is a Nef-Tat-His fusion protein and is carboxymethylated.

9. A composition as claimed in any one of claims 1 to 8, additionally comprising an adjuvant.

10. A composition as claimed in claim 9, wherein the adjuvant is a TH1 inducing : adjuvant.

11. A composition as claimed in claim 9 or 10 which adjuvant comprises monophosphoryl lipid A or a derivative thereof such as 3 de-O-acylated monophosphoryl lipid A.

12. A composition as claimed in any one of claims 9 to 11 additionally comprising a saponin adjuvant.

13. A composition as claimed in claim 11 or claim 12 which additionally comprises an oil in water emulsion and tocopherol.

14. A composition as claimed in any one of claims 9 to claim 13 wherein the adjuvant comprises 3D-MPL., QS21 and an oil in water emulsion of tocopherol, squalene and Tween 80™.

15. A composition as claimed in any one of claims 1 to 14 further comprising HIV gp160 or its derivative gp120.

16. A protein comprising an entire HIV Tat protein or entire Tat with a C terminal histidine tail, or a mutated entire Tat which has undergone deletion, addition or substitution of one amino acid, or a mutated Tat as defined by SEQ ID NO. 23, linked to an entire HIV Nef protein or entire Nef with a C- terminal histidine tail, or entire Nef which has undergone deletion, addition or substitution of one amino acid, in Nef-Tat or Tat-Nef orientation.

17. A method of preparing a protein comprising (a) an entire HIV Tat protein or Tat with a C terminal histidine tail, or a mutated Tat which has undergone deletion, addition or substitution of one amino acid, or a mutated Tat as defined by as defined by SEQ ID NO. 23, linked to either (i) a protein or lipoprotein fusion partner or (ii) an entire HIV Nef protein or Nef with a C- terminal histidine tail, or Nef which has undergone deletion, addition or substitution of one amino acid; or (b) an entire HIV Nef protein or Nef with a C- terminal histidine tail, or Nef which has undergone deletion, addition or substitution of one amino acid, linked to either (i) a protein or lipoprotein fusion partner or (ii) an entire HIV Tat protein or Tat with a C terminal histidine tail, or a mutated Tat which has undergone deletion, addition or substitution of one amino acid, or a mutated Tat as defined by SEQ ID NO. 23; or (c) an entire HIV Nef protein or Nef with a C- terminal histidine tail, or Nef which has undergone deletion, addition or substitution of one amino acid, linked to an entire HIV Tat protein or Tat with a C terminal histidine tail, or a mutated Tat which has undergone deletion, addition or substitution of one amino acid, or a mutated Tat as defined by SEQ ID NO. 23, and a protein or lipoprotein fusion partner, in *Pichia pastoris* which method comprises the steps of transforming *Pichia patoris* with DNA encoding said protein, expressing said protein and recovering the protein.

18. The method of claim 17 wherein the protein is a Nef-Tat-His fusion protein and the method further comprises a carboxymethylation step performed on the expressed protein.

19. A method of producing a vaccine, comprising admixing the protein from claims 17 and 18 with a pharmaceutically acceptable diluent.

20. The method of claim 19 further comprising the addition of HIV gp 160 or its derivative gp120.

21. The method of claims 17 to 20 further comprising the addition of an adjuvant, particularly a TH1 inducing adjuvant.

22. A Nef-Tat-His or a Nef Tat Mutant His protein or polynucleotide having the amino acid or DNA sequence shown in SEQ ID NOs. 12, 13, 16, 17, 20, 21, 24 or 25.

## Patentansprüche

1. Protein-Impfstoffzusammensetzung, die ein exprimiertes, gewonnenes und isoliertes Protein umfasst, umfassend:
(a) ein vollständiges HIV Tat-Protein oder Tat mit einem C-terminalen Histidinschwanz oder ein mutiertes Tat, das eine Deletion, Addition oder Substitution einer Aminosäure erfahren hat, oder ein mutiertes Tat wie in SEQ ID NO: 23 definiert, entweder gebunden an (i) einen Protein- oder Lipoprotein-Fusionspartner oder (ii) das vollständige HIV Nef-Protein oder Nef mit einem C-terminalen Histidinschwanz oder Nef, das eine Deletion, Addition oder Substitution einer Aminosäure erfahren hat; oder
(b) ein vollständiges HIV Nef-Protein oder Nef mit einem C-terminalen Histidinschwanz oder Nef, das eine Deletion, Addition oder Substitution einer Aminosäure erfahren hat, entweder gebunden an (i) einen Protein- oder Lipoprotein-Fusionspartner oder (ii) das vollständige HIV Tat-Protein oder Tat mit einem C-terminalen Histidinschwanz oder ein mutiertes Tat, das eine Deletion, Addition oder Substitution einer Aminosäure erfahren hat, oder ein mutiertes Tat wie in SEQ ID NO: 23 definiert; oder
(c) ein vollständiges HIV Nef-Protein oder Nef mit einem C-terminalen Histidinschwanz oder Nef, das eine Deletion, Addition oder Substitution einer Aminosäure erfahren hat, gebunden an das vollständige HIV Tat-Protein oder Tat mit einem C-terminalen Histidinschwanz oder ein mutiertes Tat, das eine Deletion, Addition oder Substitution einer Aminosäure erfahren hat, oder ein mutiertes Tat wie in SEQ ID NO: 23 definiert, und einen Protein- oder Lipoprotein-Fusionspartner,
in einer Mischung mit einem pharmazeutisch annehmbaren Exzipienten.

2. Zusammensetzung gemäß Anspruch 1, umfassend ein Tat-Nef-Fusionsprotein oder Derivat davon.

3. Zusammensetzung gemäß Anspruch 1, umfassend ein Nef-Tat-Fusionsprotein oder ein Derivat davon.

4. Zusammensetzung gemäß irgendeinem der Ansprüche 1 bis 3, wobei das Lipoprotein das Hämophilus Influenza B Protein D oder ein Derivat davon ist.

5. Zusammensetzung gemäß Anspruch 4, wobei der Fusionspartner zwischen 100 bis 130 Aminosäuren vom N-Terminus des Hämophilus Influenza B Proteins D umfaßt.

6. Zusammensetzung gemäß irgendeinem der Ansprüche 1 bis 5, wobei das Tat-Protein mit einem HIV Nef-Protein und einem Fusionspartner fusioniert ist.

7. Zusammensetzung gemäß irgendeinem der Ansprüche 1 bis 6, wobei das Protein einen Histidinschwanz hat.

8. Zusammensetzung gemäß irgendeinem der Ansprüche 1 bis 7, wobei das Protein ein Nef-Tat-His-Fusionsprotein ist und carboxymethyliert ist.

9. Zusammensetzung gemäß irgendeinem der Ansprüche 1 bis 8, zusätzlich umfassend ein Adjuvans.

10. Zusammensetzung gemäß Anspruch 9, wobei das Adjuvans ein TH1-induzierendes Adjuvans ist.

11. Zusammensetzung gemäß Anspruch 9 oder 10, wobei das Adjuvans Monophosphoryllipid A oder ein Derivat davon, wie 3-de-O-acyliertes Monophosphoryllipid A, umfaßt.

12. Zusammensetzung gemäß einem der Ansprüche 9 bis 11, zusätzlich umfassend ein Saponin-Adjuvans.

13. Zusammensetzung gemäß Anspruch 11 oder 12, welche zusätzlich eine Öl-in-Wasser-Emulsion und Tocopherol umfaßt.

14. Zusammensetzung gemäß irgendeinem der Ansprüche 9 bis 13, wobei das Adjuvans 3D-MPL, QS21 und eine Öl-in-Wasser-Emulsion von Tocopherol, Squalen und Tween 80^{™} umfaßt.

15. Zusammensetzung gemäß irgendeinem der Ansprüche 1 bis 14, weiterhin umfassend HIV gp160 oder dessen Derivat gp120.

16. Protein, das das vollständige HIV Tat-Protein oder das vollständige Tat mit einem C-terminalen Histidinschwanz oder ein mutiertes vollständiges Tat, das eine Deletion, Addition oder Substitution einer Aminosäure erfahren hat, oder ein mutiertes Tat wie in SEQ ID NO: 23 definiert, umfaßt, gebunden an das vollständige HIV Nef-Protein oder vollständiges Nef mit einem C-terminalen Histidinschwanz oder vollständiges Nef, das eine Deletion, Addition oder Substitution einer Aminosäure erfahren hat, in Nef-Tat- oder Tat-Nef-Orientierung.

17. Verfahren zum Herstellen eines Proteins umfassend (a) ein vollständiges HIV Tat-Protein oder Tat mit einem C-terminalen Histidinschwanz oder ein mutiertes Tat, das eine Deletion, Addition oder Substitution einer Aminosäure erfahren hat, oder ein mutiertes Tat wie in SEQ ID NO: 23 definiert, entweder gebunden an (i) einen Protein- oder Lipoprotein-Fusionspartner oder (ii) das vollständige HIV Nef-Protein oder Nef mit einem C-terminalen Histidinschwanz oder Nef, das eine Deletion, Addition oder Substitution einer Aminosäure erfahren hat; oder (b) ein vollständiges HIV Nef-Protein oder Nef mit einem C-terminalen Histidinschwanz oder Nef, das eine Deletion, Addition oder Substitution einer Aminosäure erfahren hat, entweder gebunden an (i) einen Protein- oder Lipoprotein-Fusionspartner oder (ii) ein vollständiges HIV Tat-Protein oder Tat mit einem C-terminalen Histidinschwanz oder ein mutiertes Tat, das eine Deletion, Addition oder Substitution einer Aminosäure erfahren hat, oder ein mutiertes Tat wie in SEQ ID NO: 23 definiert; oder (c) ein vollständiges HIV Nef-Protein oder Nef mit einem C-terminalen Histidinschwanz oder Nef, das eine Deletion, Addition oder Substitution einer Aminosäure erfahren hat, gebunden an ein vollständiges HIV Tat-Protein oder Tat mit einem C-terminalen Histidinschwanz oder ein mutiertes Tat, das eine Deletion, Addition oder Substitution einer Aminosäure erfahren hat, oder ein mutiertes Tat wie in SEQ ID NO: 23 definiert, und einen Protein- oder Lipoprotein-Fusionspartner; in Pichia pastoris, wobei das Verfahren die Schritte der Transformierung von Pichia pastoris mit DNA, die das Protein codiert, Exprimieren des Proteins und Gewinnen des Proteins umfaßt.

18. Verfahren gemäß Anspruch 17, wobei das Protein ein Nef-Tat-His-Fusionsprotein ist, wobei das Verfahren weiterhin einen Carboxymethylierungsschritt umfaßt, der an dem exprimierten Protein durchgeführt wird.

19. Verfahren zum Herstellen eines Impfstoffs, umfassend Mischen des Proteins gemäß Anspruch 17 und 18 mit einem pharmazeutisch annehmbaren Verdünnungsmittel.

20. Verfahren gemäß Anspruch 19, weiterhin umfassend die Zugabe von HIV gp160 oder dessen Derivat gp120.

21. Verfahren gemäß Anspruch 17 bis 20, weiterhin umfassend die Zugabe eines Adjuvans, insbesondere eines TH1-induzierenden Adjuvans.

22. Net-Tat-His oder Nef-Tat-Mutanten-His-Protein oder Polynukleotid mit der Aminosäure oder DNA-Sequenz, die in den SEQ ID NOs: 12, 13, 16, 17, 20, 21, 24 oder 25 gezeigt ist.

## Revendications

1. Composition de vaccin protéinique qui comprend une protéine exprimée, récupérée et isolée comprenant :
(a) une protéine Tat entière du VIH ou une protéine Tat avec une queue histidine en C-terminal, ou une protéine Tat mutée qui a subi une délétion, addition ou substitution d'un acide aminé, ou une protéine Tat mutée telle que définie par la SEQ ID NO : 23, liée soit à (i) une protéine ou un partenaire de fusion de lipoprotéine, soit à (ii) une protéine Nef entière du VIH ou une protéine Nef avec une queue histidine en C-terminal, ou une protéine Nef qui a subi une délétion, addition ou substitution d'un acide aminé ; ou
(b) une protéine Nef entière du VIH ou une protéine Nef avec une queue histidine en C-terminal, ou une protéine Nef qui a subi une délétion, addition ou substitution d'un acide aminé, liée soit à (i) une protéine ou un partenaire de fusion de lipoprotéine, soit à (ii) une protéine Tat entière du VIH ou une protéine Tat avec une queue histidine en C-terminal, ou une protéine Tat mutée qui a subi une délétion, addition ou substitution d'un acide aminé, ou une protéine Tat mutée telle que définie par la SEQ ID NO : 23 ; ou
(c) une protéine Nef entière du VIH ou une protéine Nef avec une queue histidine en C-terminal, ou une protéine Nef qui a subi une délétion, addition ou substitution d'un acide aminé, liée à une protéine Tat entière du VIH ou une protéine Tat avec une queue histidine en C-terminal, ou une protéine Tat mutée qui a subi une délétion, addition ou substitution d'un acide aminé, ou une protéine Tat mutée telle que définie par la SEQ ID NO : 23, et une protéine ou un partenaire de fusion de lipoprotéine,
dans un mélange avec un excipient pharmaceutiquement acceptable.

2. Composition telle que définie dans la revendication 1 comprenant une protéine de fusion Tat-Nef ou un dérivé de celle-ci.

3. Composition telle que définie dans la revendication 1, comprenant une protéine de fusion Nef-Tat ou un dérivé de celle-ci.

4. Composition telle que définie dans l'une quelconque des revendications 1 à 3, dans laquelle la lipoprotéine est la protéine D d'Haemophilus Influenza B ou un dérivé de celle-ci.

5. Composition telle que définie dans la revendication 4, dans laquelle le partenaire de fusion comprend entre 100 et 130 acides aminés du N-terminal de la protéine D d'Haemophilus Influenza B.

6. Composition telle que définie dans l'une quelconque des revendications 1 à 5, dans laquelle la protéine Tat est fusionnée à une protéine Nef du VIH et à un partenaire de fusion.

7. Composition telle que définie dans l'une quelconque des revendications 1 à 6, dans laquelle la protéine a une queue histidine.

8. Composition telle que définie dans l'une quelconque des revendications 1 à 7, dans laquelle la protéine est une protéine de fusion Nef-Tat-His et est carboxyméthylée.

9. Composition telle que définie dans l'une quelconque des revendications 1 à 8, comprenant en outre un adjuvant.

10. Composition telle que définie dans la revendication 9, dans laquelle l'adjuvant est un adjuvant induisant TH1

11. Composition telle que définie dans la revendication 9 ou 10, dans laquelle l'adjuvant comprend un monophosphoryl-lipide A ou un dérivé de celui-ci tel qu'un monophosphoryl-lipide A 3-dé-O-acylé.

12. Composition telle que définie dans l'une quelconque des revendications 9 à 11, comprenant en outre un adjuvant à base de saponine.

13. Composition telle que définie dans la revendication 11 ou la revendication 12 qui comprend en outre une émulsion d'huile dans l'eau et du tocophérol.

14. Composition telle que définie dans l'une quelconque des revendications 9 à 13, dans laquelle l'adjuvant comprend du 3D-MPL, du QS21 et une émulsion d'huile dans l'eau de tocophérol, squalène et Tween 80™_{.}

15. Composition telle que définie dans l'une quelconque des revendications 1 à 14, comprenant en outre gp160 du VIH ou son dérivé gp120.

16. Protéine comprenant une protéine Tat entière du VIH ou une protéine Tat entière avec une queue histidine en C-terminal, ou une protéine Tat entière mutée qui a subi une délétion, addition ou substitution d'un acide aminé, ou une protéine Tat mutée telle que définie par la SEQ ID NO : 23, liée à une protéine Nef entière du VIH ou une protéine Nef entière avec une queue histidine en C-terminal, ou une protéine Nef entière qui a subi une délétion, addition ou substitution d'un acide aminé, dans une orientation Nef-Tat ou Tat-Nef.

17. Procédé de préparation d'une protéine comprenant (a) une protéine Tat entière du VIH ou une protéine Tat avec une queue histidine en C-terminal, ou une protéine Tat mutée qui a subi une délétion, addition ou substitution d'un acide aminé, ou une protéine Tat mutée telle que définie par la SEQ ID NO : 23, liée soit à (i) une protéine ou un partenaire de fusion de lipoprotéine, soit à (ii) une protéine Nef entière du VIH ou une protéine Nef avec une queue histidine en C-terminal, ou une protéine Nef qui a subi une délétion, addition ou substitution d'un acide aminé ; ou (b) une protéine Nef entière du VIH ou une protéine Nef avec une queue histidine en C-terminal, ou une protéine Nef qui a subi une délétion, addition ou substitution d'un acide aminé, liée soit à (i) une protéine ou un partenaire de fusion de lipoprotéine, soit à (ii) une protéine Tat entière du VIH ou une protéine Tat avec une queue histidine en C-terminal, ou une protéine Tat mutée qui a subi une délétion, addition ou substitution d'un acide aminé, ou une protéine Tat mutée telle que définie par la SEQ ID NO : 23 ; ou (c) une protéine Nef entière du VIH ou une protéine Nef avec une queue histidine en C-terminal, ou une protéine Nef qui a subi une délétion, addition ou substitution d'un acide aminé, liée à une protéine Tat entière du VIH ou une protéine Tat avec une queue histidine en C-terminal, ou une protéine Tat mutée qui a subi une délétion, addition ou substitution d'un acide aminé, ou une protéine Tat mutée telle que définie par la SEQ ID NO : 23, et une protéine ou un partenaire de fusion de lipoprotéine, dans Pichia *pastoris,* lequel procédé comprend les étapes de transformation de *Pichia pastoris* avec un ADN codant pour ladite protéine, d'expression de ladite protéine et de récupération de la protéine.

18. Procédé selon la revendication 17, dans lequel la protéine est une protéine de fusion Nef-Tat T-His et le procédé comprend en outre une étape de carboxyméthylation exécutée sur la protéine exprimée.

19. Procédé de production d'un vaccin, comprenant le mélange de la protéine selon les revendications 17 et 18 avec un diluant pharmaceutiquement acceptable.

20. Procédé selon la revendication 19, comprenant en outre l'addition de gp160 du VIH ou son dérivé gp120.

21. Procédé selon les revendications 17 à 20, comprenant en outre l'addition d'un adjuvant, en particulier d'un adjuvant induisant TH1.

22. Protéine Nef-Tat-His ou Nef-Tat-Mutant-His ou polynucléotide ayant la séquence d'acides aminés ou d'ADN représentée par les SEQ ID NO : 12, 13, 16, 17, 20, 21, 24 ou 25.


### SEQUENCE LISTING

(1) GENERAL INFORMATION
   (i) APPLICANT: SmithKline Beecham Biologicals S.A.
   (ii) TITLE OF THE INVENTION: Vaccine
   (iii) NUMBER OF SEQUENCES: 27
   (iv) CORRESPONDENCE ADDRESS:
      (A) ADDRESSEE: SmithKline Beecham
      (B) STREET: Two New Horizons Court
      (C) CITY: Brentford
      (D) STATE:
      (E) COUNTRY: Middx, UK
      (F) ZIP: TW8 9EP
   (v) COMPUTER READABLE FORM:
      (A) MEDIUM TYPE: Diskette
      (B) COMPUTER: IBM Compatible
      (C) OPERATING SYSTEM: DOS
      (D) SOFTWARE: FastSEQ for Windows Version 2.0
   (vi) CURRENT APPLICATION DATA:
      (A) APPLICATION NUMBER:
      (B) FILING DATE: 26-SEP-1997
      (C) CLASSIFICATION:
   (vii) PRIOR APPLICATION DATA:
      (A) APPLICATION NUMBER:
      (B) FILING DATE:
   (viii) ATTORNEY/AGENT INFORMATION:
      (A) NAME: Bor, Fiona R
      (B) REGISTRATION NUMBER:
      (C) REFERENCE/DOCKET NUMBER:
   (ix) TELECOMMUNICATION INFORMATION:
      (A) TELEPHONE: 0181 975 2817
      (B) TELEFAX: 0181 975 6141
      (C) TELEX:
(2) INFORMATION FOR SEQ ID NO:1:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO:1:
      ATCGTCCATG .GGT.GGC.A AG.TGG.T 28
(2) INFORMATION FOR SEQ ID NO:2:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:2:
      CGGCTACTAG TGCAGTTCTT GAA 23
(2) INFORMATION FOR SEQ ID NO:3:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:3:
      ATCGTACTAG T.GAG.CCA. GTA.GAT.C 29
(2) INFORMATION FOR SEQ ID NO:4:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:4:
      CGGCTACTAG TTTCCTTCGG GCCT 24
(2) INFORMATION FOR SEQ ID NO:5:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (xi) SEQUENCE DESCRIPTION: SEQ ID NO:5:
      ATCGTCCATG GAGCCAGTAG ATC 23
(2) INFORMATION FOR SEQ ID NO:6:
   (i) SEQUENCE CHARACTERISTICS:
      (A) LENGTH: 441 base pairs
      (B) TYPE: nucleic acid
      (C) STRANDEDNESS: single
      (D) TOPOLOGY: linear
   (xi) SEQUENCE DESCRIPTION: SEQ ID NO:6:
   ATGGATCCAA AAACTTTAGC CCTTTCTTTA TTAGCAGCTG GCGTACTAGC AGGTTGTAGC    60
   AGCCATTCAT CAAATATGGC GAATACCCAA ATGAAATCAG ACAAAATCAT TATTGCTCAC   120
   CGTGGTGCTA GCGGTTATTT ACCAGAGCAT ACGTTAGAAT CTAAAGCACT TGCTTTTGCA   180
   CAACAGGCTG ATTATTTAGA GCAAGATTTA GCAATGACTA AGGATGGTCG TTTAGTGGTT   240
   ATTCACGATC ACTTTTTAGA TGGCTTGACT GATGTTGCGA AAAAATTCCC ACATCGTCAT   300
   CGTAAAGATG GCCGTTACTA TGTCATCGAC TTTACCTTAA AAGAAATTCA AAGTTTAGAA   360
   ATGACAGAAA ACTTTGAAAC CATGGCCACG TGTGATCAGA GCTCAACTAG TGGCCACCAT   420
   CACCATCACC ATTAATCTAG A                                             441
(2) INFORMATION FOR SEQ ID NO:7:
   (i) SEQUENCE CHARACTERISTICS:
      (A) LENGTH: 144 amino acids
      (B) TYPE: amino acid
      (C) STRANDEDNESS: single
      (D) TOPOLOGY: linear
   (xi) SEQUENCE DESCRIPTION: SEQ ID NO:7:
   Met Asp Pro Lys Thr Leu Ala Leu Ser Leu Leu Ala Ala Gly Val Leu
    1               5                  10                  15
   Ala Gly Cys Ser Ser His Ser Ser Asn Met Ala Asn Thr Gln Met Lys
               20                  25                  30
   Ser Asp Lys Ile Ile Ile Ala His Arg Gly Ala Ser Gly Tyr Leu Pro
           35                  40                  45
   Glu His Thr Leu Glu Ser Lys Ala Leu Ala Phe Ala Gln Gln Ala Asp
       50                  55                  60
   Tyr Leu Glu Gln Asp Leu Ala Met Thr Lys Asp Gly Arg Leu Val Val
   65                  70                  75                  80
   Ile His Asp His Phe Leu Asp Gly Leu Thr Asp Val Ala Lys Lys Phe
                   85                  90                  95
   Pro His Arg His Arg Lys Asp Gly Arg Tyr Tyr Val Ile Asp Phe Thr
               100                 105                 110
   Leu Lys Glu Ile Gln Ser Leu Glu Met Thr Glu Asn Phe Glu Thr Met
           115                 120                 125
   Ala Thr Cys Asp Gln Ser Ser Thr Ser Gly His His His His His His
       130                 135                 140
(2) INFORMATION FOR SEQ ID NO:8:
   (i) SEQUENCE CHARACTERISTICS:
      (A) LENGTH: 648 base pairs
      (B) TYPE: nucleic acid
      (C) STRANDEDNESS: single
      (D) TOPOLOGY: linear
   (xi) SEQUENCE DESCRIPTION: SEQ ID NO:8:
   ATGGGTGGCA AGTGGTCAAA AAGTAGTGTG GTTGGATGGC CTACTGTAAG GGAAAGAATG    60
   AGACGAGCTG AGCCAGCAGC AGATGGGGTG GGAGCAGCAT CTCGAGACCT GGAAAAACAT   120
   GGAGCAATCA CAAGTAGCAA TACAGCAGCT ACCAATGCTG CTTGTGCCTG GCTAGAAGCA   180
   CAAGAGGAGG AGGAGGTGGG TTTTCCAGTC ACACCTCAGG TACCTTTAAG ACCAATGACT   240
   TACAAGGCAG CTGTAGATCT TAGCCACTTT TTAAAAGAAA AGGGGGGACT GGAAGGGCTA   300
   ATTCACTCCC AACGAAGACA AGATATCCTT GATCTGTGGA TCTACCACAC ACAAGGCTAC   360
   TTCCCTGATT GGCAGAACTA CACACCAGGG CCAGGGGTCA GATATCCACT GACCTTTGGA   420
   TGGTGCTACA AGCTAGTACC AGTTGAGCCA GATAAGGTAG AAGAGGCCAA TAAAGGAGAG   480
   AACACCAGCT TGTTACACCC TGTGAGCCTG CATGGAATGG ATGACCCTGA GAGAGAAGTG   540
   TTAGAGTGGA GGTTTGACAG CCGCCTAGCA TTTCATCACG TGGCCCGAGA GCTGCATCCG   600
   GAGTACTTCA AGAACTGCAC TAGTGGCCAC CATCACCATC ACCATTAA                648
(2) INFORMATION FOR SEQ ID NO:9:
   (i) SEQUENCE CHARACTERISTICS:
      (A) LENGTH: 216 amino acids
      (B) TYPE: amino acid
      (C) STRANDEDNESS: single
      (D) TOPOLOGY: linear
   (xi) SEQUENCE DESCRIPTION: SEQ ID NO:9:
(2) INFORMATION FOR SEQ ID NO:10:
   (i) SEQUENCE CHARACTERISTICS:
      (A) LENGTH: 288 base pairs
      (B) TYPE: nucleic acid
      (C) STRANDEDNESS: single
      (D) TOPOLOGY: linear
   (xi) SEQUENCE DESCRIPTION: SEQ ID NO:10:
   ATGGAGCCAG TAGATCCTAG ACTAGAGCCC TGGAAGCATC CAGGAAGTCA GCCTAAAACT    60
   GCTTGTACCA ATTGCTATTG TAAAAAGTGT TGCTTTCATT GCCAAGTTTG TTTCATAACA   120
   AAAGCCTTAG GCATCTCCTA TGGCAGGAAG AAGCGGAGAC AGCGACGAAG ACCTCCTCAA   180
   GGCAGTCAGA CTCATCAAGT TTCTCTATCA AAGCAACCCA CCTCCCAATC CCGAGGGGAC   240
   CCGACAGGCC CGAAGGAAAC TAGTGGCCAC CATCACCATC ACCATTAA                288
(2) INFORMATION FOR SEQ ID NO:11:
   (i) SEQUENCE CHARACTERISTICS:
      (A) LENGTH: 96 amino acids
      (B) TYPE: amino acid
      (C) STRANDEDNESS: single
      (D) TOPOLOGY: linear
   (xi) SEQUENCE DESCRIPTION: SEQ ID NO:11:
(2) INFORMATION FOR SEQ ID NO:12:
   (i) SEQUENCE CHARACTERISTICS:
      (A) LENGTH: 909 base pairs
      (B) TYPE: nucleic acid
      (C) STRANDEDNESS: single
      (D) TOPOLOGY: linear
   (xi) SEQUENCE DESCRIPTION: SEQ ID NO:12:
   ATGGGTGGCA AGTGGTCAAA AAGTAGTGTG GTTGGATGGC CTACTGTAAG GGAAAGAATG    60
   AGACGAGCTG AGCCAGCAGC AGATGGGGTG GGAGCAGCAT CTCGAGACCT GGAAAAACAT   120
   GGAGCAATCA CAAGTAGCAA TACAGCAGCT ACCAATGCTG CTTGTGCCTG GCTAGAAGCA   180
   CAAGAGGAGG AGGAGGTGGG TTTTCCAGTC ACACCTCAGG TACCTTTAAG ACCAATGACT   240
   TACAAGGCAG CTGTAGATCT TAGCCACTTT TTAAAAGAAA AGGGGGGACT GGAAGGGCTA   300
   ATTCACTCCC AACGAAGACA AGATATCCTT GATCTGTGGA TCTACCACAC ACAAGGCTAC   360
   TTCCCTGATT GGCAGAACTA CACACCAGGG CCAGGGGTCA GATATCCACT GACCTTTGGA   420
   TGGTGCTACA AGCTAGTACC AGTTGAGCCA GATAAGGTAG AAGAGGCCAA TAAAGGAGAG   480
   AACACCAGCT TGTTACACCC TGTGAGCCTG CATGGAATGG ATGACCCTGA GAGAGAAGTG   540
   TTAGAGTGGA GGTTTGACAG CCGCCTAGCA TTTCATCACG TGGCCCGAGA GCTGCATCCG   600
   GAGTACTTCA AGAACTGCAC TAGTGAGCCA GTAGATCCTA GACTAGAGCC CTGGAAGCAT   660
   CCAGGAAGTC AGCCTAAAAC TGCTTGTACC AATTGCTATT GTAAAAAGTG TTGCTTTCAT   720
   TGCCAAGTTT GTTTCATAAC AAAAGCCTTA GGCATCTCCT ATGGCAGGAA GAAGCGGAGA   780
   CAGCGACGAA GACCTCCTCA AGGCAGTCAG ACTCATCAAG TTTCTCTATC AAAGCAACCC   840
   ACCTCCCAAT CCCGAGGGGA CCCGACAGGC CCGAAGGAAA CTAGTGGCCA CCATCACCAT   900
   CACCATTAA                                                           909
(2) INFORMATION FOR SEQ ID NO:13:
   (i) SEQUENCE CHARACTERISTICS:
      (A) LENGTH: 303 amino acids
      (B) TYPE: amino acid
      (C) STRANDEDNESS: single
      (D) TOPOLOGY: linear
   (xi) SEQUENCE DESCRIPTION: SEQ ID NO:13:
(2) INFORMATION FOR SEQ ID NO:14:
   (i) SEQUENCE CHARACTERISTICS:
      (A) LENGTH: 1029 base pairs
      (B) TYPE: nucleic acid
      (C) STRANDEDNESS: single
      (D) TOPOLOGY: linear
   (xi) SEQUENCE DESCRIPTION: SEQ ID NO:14:
   ATGGATCCAA AAACTTTAGC CCTTTCTTTA TTAGCAGCTG GCGTACTAGC AGGTTGTAGC     60
   AGCCATTCAT CAAATATGGC GAATACCCAA ATGAAATCAG ACAAAATCAT TATTGCTCAC    120
   CGTGGTGCTA GCGGTTATTT ACCAGAGCAT ACGTTAGAAT CTAAAGCACT TGCTTTTGCA    180
   CAACAGGCTG ATTATTTAGA GCAAGATTTA GCAATGACTA AGGATGGTCG TTTAGTGGTT    240
   ATTCACGATC ACTTTTTAGA TGGCTTGACT GATGTTGCGA AAAAATTCCC ACATCGTCAT    300
   CGTAAAGATG GCCGTTACTA TGTCATCGAC TTTACCTTAA AAGAAATTCA AAGTTTAGAA    360
   ATGACAGAAA ACTTTGAAAC CATGGGTGGC AAGTGGTCAA AAAGTAGTGT GGTTGGATGG    420
   CCTACTGTAA GGGAAAGAAT GAGACGAGCT GAGCCAGCAG CAGATGGGGT GGGAGCAGCA    480
   TCTCGAGACC TGGAAAAACA TGGAGCAATC ACAAGTAGCA ATACAGCAGC TACCAATGCT    540
   GCTTGTGCCT GGCTAGAAGC ACAAGAGGAG GAGGAGGTGG GTTTTCCAGT CACACCTCAG    600
   GTACCTTTAA GACCAATGAC TTACAAGGCA GCTGTAGATC TTAGCCACTT TTTAAAAGAA    660
   AAGGGGGGAC TGGAAGGGCT AATTCACTCC CAACGAAGAC AAGATATCCT TGATCTGTGG    720
   ATCTACCACA CACAAGGCTA CTTCCCTGAT TGGCAGAACT ACACACCAGG GCCAGGGGTC    780
   AGATATCCAC TGACCTTTGG ATGGTGCTAC AAGCTAGTAC CAGTTGAGCC AGATAAGGTA    840
   GAAGAGGCCA ATAAAGGAGA GAACACCAGC TTGTTACACC CTGTGAGCCT GCATGGAATG    900
   GATGACCCTG AGAGAGAAGT GTTAGAGTGG AGGTTTGACA GCCGCCTAGC ATTTCATCAC    960
   GTGGCCCGAG AGCTGCATCC GGAGTACTTC AAGAACTGCA CTAGTGGCCA CCATCACCAT   1020
   CACCATTAA                                                           1029
(2) INFORMATION FOR SEQ ID NO:15:
   (i) SEQUENCE CHARACTERISTICS:
      (A) LENGTH: 325 amino acids
      (B) TYPE: amino acid
      (C) STRANDEDNESS: single
      (D) TOPOLOGY: linear
   (xi) SEQUENCE DESCRIPTION: SEQ ID NO:15:
(2) INFORMATION FOR SEQ ID NO: 16:
   (i) SEQUENCE CHARACTERISTICS:
      (A) LENGTH: 1290 base pairs
      (B) TYPE: nucleic acid
      (C) STRANDEDNESS: single
      (D) TOPOLOGY: linear
   (xi) SEQUENCE DESCRIPTION: SEQ ID NO:16:
   ATGGATCCAA AAACTTTAGC CCTTTCTTTA TTAGCAGCTG GCGTACTAGC AGGTTGTAGC     60
   AGCCATTCAT CAAATATGGC GAATACCCAA ATGAAATCAG ACAAAATCAT TATTGCTCAC    120
   CGTGGTGCTA GCGGTTATTT ACCAGAGCAT ACGTTAGAAT CTAAAGCACT TGCGTTTGCA    180
   CAACAGGCTG ATTATTTAGA GCAAGATTTA GCAATGACTA AGGATGGTCG TTTAGTGGTT    240
   ATTCACGATC ACTTTTTAGA TGGCTTGACT GATGTTGCGA AAAAATTCCC ACATCGTCAT    300
   CGTAAAGATG GCCGTTACTA TGTCATCGAC TTTACCTTAA AAGAAATTCA AAGTTTAGAA    360
   ATGACAGAAA ACTTTGAAAC CATGGGTGGC AAGTGGTCAA AAAGTAGTGT GGTTGGATGG    420
   CCTACTGTAA GGGAAAGAAT GAGACGAGCT GAGCCAGCAG CAGATGGGGT GGGAGCAGCA    480
   TCTCGAGACC TGGAAAAACA TGGAGCAATC ACAAGTAGCA ATACAGCAGC TACCAATGCT    540
   GCTTGTGCCT GGCTAGAAGC ACAAGAGGAG GAGGAGGTGG GTTTTCCAGT CACACCTCAG    600
   GTACCTTTAA GACCAATGAC TTACAAGGCA GCTGTAGATC TTAGCCACTT TTTAAAAGAA    660
   AAGGGGGGAC TGGAAGGGCT AATTCACTCC CAACGAAGAC AAGATATCCT TGATCTGTGG    720
   ATCTACCACA CACAAGGCTA CTTCCCTGAT TGGCAGAACT ACACACCAGG GCCAGGGGTC    780
   AGATATCCAC TGACCTTTGG ATGGTGCTAC AAGCTAGTAC CAGTTGAGCC AGATAAGGTA    840
   GAAGAGGCCA ATAAAGGAGA GAACACCAGC TTGTTACACC CTGTGAGCCT GCATGGAATG    900
   GATGACCCTG AGAGAGAAGT GTTAGAGTGG AGGTTTGACA GCCGCCTAGC ATTTCATCAC    960
   GTGGCCCGAG AGCTGCATCC GGAGTACTTC AAGAACTGCA CTAGTGAGCC AGTAGATCCT   1020
   AGACTAGAGC CCTGGAAGCA TCCAGGAAGT CAGCCTAAAA CTGCTTGTAC CAATTGCTAT   1080
   TGTAAAAAGT GTTGCTTTCA TTGCCAAGTT TGTTTCATAA CAAAAGCCTT AGGCATCTCC   1140
   TATGGCAGGA AGAAGCGGAG ACAGCGACGA AGACCTCCTC AAGGCAGTCA GACTCATCAA   1200
   GTTTCTCTAT CAAAGCAACC CACCTCCCAA TCCCGAGGGG ACCCGACAGG CCCGAAGGAA   1260
   ACTAGTGGCC ACCATCACCA TCACCATTAA                                    1290
(2) INFORMATION FOR SEQ ID NO:17:
   (i) SEQUENCE CHARACTERISTICS:
      (A) LENGTH: 412 amino acids
      (B) TYPE: amino acid
      (C) STRANDEDNESS: single
      (D) TOPOLOGY: linear
   (xi) SEQUENCE DESCRIPTION: SEQ ID NO:17:
(2) INFORMATION FOR SEQ ID NO:18:
   (i) SEQUENCE CHARACTERISTICS:
      (A) LENGTH: 981 base pairs
      (B) TYPE: nucleic acid
      (C) STRANDEDNESS: single
      (D) TOPOLOGY: linear
   (xi) SEQUENCE DESCRIPTION: SEQ ID NO:18:
(2) INFORMATION FOR SEQ ID NO:19:
   (i) SEQUENCE CHARACTERISTICS:
      (A) LENGTH: 327 amino acids
      (B) TYPE: amino acid
      (C) STRANDEDNESS: single
      (D) TOPOLOGY: linear
   (xi) SEQUENCE DESCRIPTION: SEQ ID NO:19:
(2) INFORMATION FOR SEQ ID NO:20:
   (i) SEQUENCE CHARACTERISTICS:
      (A) LENGTH: 1242 base pairs
      (B) TYPE: nucleic acid
      (C) STRANDEDNESS: single
      (D) TOPOLOGY: linear
   (xi) SEQUENCE DESCRIPTION: SEQ ID NO:20:
   ATGGATCCAA GCAGCCATTC ATCAAATATG GCGAATACCC AAATGAAATC AGACAAAATC    60
   ATTATTGCTC ACCGTGGTGC TAGCGGTTAT TTACCAGAGC ATACGTTAGA ATCTAAAGCA   120
   CTTGCGTTTG CACAACAGGC TGATTATTTA GAGCAAGATT TAGCAATGAC TAAGGATGGT   180
   CGTTTAGTGG TTATTCACGA TCACTTTTTA GATGGCTTGA CTGATGTTGC GAAAAAATTC   240
   CCACATCGTC ATCGTAAAGA TGGCCGTTAC TATGTCATCG ACTTTACCTT AAAAGAAATT   300
   CAAAGTTTAG AAATGACAGA AAACTTTGAA ACCATGGGTG GCAAGTGGTC AAAAAGTAGT    360
   GTGGTTGGAT GGCCTACTGT AAGGGAAAGA ATGAGACGAG CTGAGCCAGC AGCAGATGGG    420
   GTGGGAGCAG CATCTCGAGA CCTGGAAAAA CATGGAGCAA TCACAAGTAG CAATACAGCA    480
   GCTACCAATG CTGCTTGTGC CTGGCTAGAA GCACAAGAGG AGGAGGAGGT GGGTTTTCCA    540
   GTCACACCTC AGGTACCTTT AAGACCAATG ACTTACAAGG CAGCTGTAGA TCTTAGCCAC    600
   TTTTTAAAAG AAAAGGGGGG ACTGGAAGGG CTAATTCACT CCCAACGAAG ACAAGATATC    660
   CTTGATCTGT GGATCTACCA CACACAAGGC TACTTCCCTG ATTGGCAGAA CTACACACCA    720
   GGGCCAGGGG TCAGATATCC ACTGACCTTT GGATGGTGCT ACAAGCTAGT ACCAGTTGAG    780
   CCAGATAAGG TAGAAGAGGC CAATAAAGGA GAGAACACCA GCTTGTTACA CCCTGTGAGC    840
   CTGCATGGAA TGGATGACCC TGAGAGAGAA GTGTTAGAGT GGAGGTTTGA CAGCCGCCTA    900
   GCATTTCATC ACGTGGCCCG AGAGCTGCAT CCGGAGTACT TCAAGAACTG CACTAGTGAG    960
   CCAGTAGATC CTAGACTAGA GCCCTGGAAG CATCCAGGAA GTCAGCCTAA AACTGCTTGT   1020
   ACCAATTGCT ATTGTAAAAA GTGTTGCTTT CATTGCCAAG TTTGTTTCAT AACAAAAGCC   1080
   TTAGGCATCT CCTATGGCAG GAAGAAGCGG AGACAGCGAC GAAGACCTCC TCAAGGCAGT   1140
   CAGACTCATC AAGTTTCTCT ATCAAAGCAA CCCACCTCCC AATCCCGAGG GGACCCGACA   1200
   GGCCCGAAGG AAACTAGTGG CCACCATCAC CATCACCATT AA                      1242
(2) INFORMATION FOR SEQ ID NO:21:
   (i) SEQUENCE CHARACTERISTICS:
      (A) LENGTH: 414 amino acids
      (B) TYPE: amino acid
      (C) STRANDEDNESS: single
      (D) TOPOLOGY: linear
   (xi) SEQUENCE DESCRIPTION: SEQ ID NO:21:
(2) INFORMATION FOR SEQ ID NO:22:
   (i) SEQUENCE CHARACTERISTICS:
      (A) LENGTH: 288 base pairs
      (B) TYPE: nucleic acid
      (C) STRANDEDNESS: single
      (D) TOPOLOGY: linear
   (xi) SEQUENCE DESCRIPTION: SEQ ID NO:22:
   ATGGAGCCAG TAGATCCTAG ACTAGAGCCC TGGAAGCATC CAGGAAGTCA GCCTAAAACT    60
   GCTTGTACCA ATTGCTATTG TAAAAAGTGT TGCTTTCATT GCCAAGTTTG TTTCATAACA   120
   GCTGCCTTAG GCATCTCCTA TGGCAGGAAG AAGCGGAGAC AGCGACGAAG ACCTCCTCAA   180
   GGCAGTCAGA CTCATCAAGT TTCTCTATCA AAGCAACCCA CCTCCCAATC CAAAGGGGAG   240
   CCGACAGGCC CGAAGGAAAC TAGTGGCCAC CATCACCATC ACCATTAA                288
(2) INFORMATION FOR SEQ ID NO:23:
   (i) SEQUENCE CHARACTERISTICS:
      (A) LENGTH: 96 amino acids
      (B) TYPE: amino acid
      (C) STRANDEDNESS: single
      (D) TOPOLOGY: linear
   (xi) SEQUENCE DESCRIPTION: SEQ ID NO:23:
(2) INFORMATION FOR SEQ ID NO:24:
   (i) SEQUENCE CHARACTERISTICS:
      (A) LENGTH: 909 base pairs
      (B) TYPE: nucleic acid
      (C) STRANDEDNESS: single
      (D) TOPOLOGY: linear
   (xi) SEQUENCE DESCRIPTION: SEQ ID NO:24:
   ATGGGTGGCA AGTGGTCAAA AAGTAGTGTG GTTGGATGGC CTACTGTAAG GGAAAGAATG    60
   AGACGAGCTG AGCCAGCAGC AGATGGGGTG GGAGCAGCAT CTCGAGACCT GGAAAAACAT   120
   GGAGCAATCA CAAGTAGCAA TACAGCAGCT ACCAATGCTG CTTGTGCCTG GCTAGAAGCA   180
   CAAGAGGAGG AGGAGGTGGG TTTTCCAGTC ACACCTCAGG TACCTTTAAG ACCAATGACT   240
   TACAAGGCAG CTGTAGATCT TAGCCACTTT TTAAAAGAAA AGGGGGGACT GGAAGGGCTA   300
   ATTCACTCCC AACGAAGACA AGATATCCTT GATCTGTGGA TCTACCACAC ACAAGGCTAC   360
   TTCCCTGATT GGCAGAACTA CACACCAGGG CCAGGGGTCA GATATCCACT GACCTTTGGA   420
   TGGTGCTACA AGCTAGTACC AGTTGAGCCA GATAAGGTAG AAGAGGCCAA TAAAGGAGAG   480
   AACACCAGCT TGTTACACCC TGTGAGCCTG CATGGAATGG ATGACCCTGA GAGAGAAGTG   540
   TTAGAGTGGA GGTTTGACAG CCGCCTAGCA TTTCATCACG TGGCCCGAGA GCTGCATCCG   600
   GAGTACTTCA AGAACTGCAC TAGTGAGCCA GTAGATCCTA GACTAGAGCC CTGGAAGCAT   660
   CCAGGAAGTC AGCCTAAAAC TGCTTGTACC AATTGCTATT GTAAAAAGTG TTGCTTTCAT   720
   TGCCAAGTTT GTTTCATAAC AGCTGCCTTA GGCATCTCCT ATGGCAGGAA GAAGCGGAGA   780
   CAGCGACGAA GACCTCCTCA AGGCAGTCAG ACTCATCAAG TTTCTCTATC AAAGCAACCC   840
   ACCTCCCAAT CCAAAGGGGA GCCGACAGGC CCGAAGGAAA CTAGTGGCCA CCATCACCAT   900
   CACCATTAA                                                           909
(2) INFORMATION FOR SEQ ID NO:25:
   (i) SEQUENCE CHARACTERISTICS:
      (A) LENGTH: 303 amino acids
      (B) TYPE: amino acid
      (C) STRANDEDNESS: single
      (D) TOPOLOGY: linear
   (xi) SEQUENCE DESCRIPTION: SEQ ID NO:25:
(2) INFORMATION FOR SEQ ID NO:26:
   (i) SEQUENCE CHARACTERISTICS:
      (A) LENGTH: 57 base pairs
      (B) TYPE: nucleic acid
      (C) STRANDEDNESS: single
      (D) TOPOLOGY: linear
   (xi) SEQUENCE DESCRIPTION: SEQ ID NO:26:
      TTCGAAACCA TGGCCGCGGA CTAGTGGCCA CCATCACCAT CACCATTAAC GGAATTC 57
(2) INFORMATION FOR SEQ ID NO:27:
   (i) SEQUENCE CHARACTERISTICS:
      (A) LENGTH: 17 amino acids
      (B) TYPE: amino acid
      (C) STRANDEDNESS: single
      (D) TOPOLOGY: linear
   (xi) SEQUENCE DESCRIPTION: SEQ ID NO: 27: